# EUROPEAN PATENT APPLICATION

(11) **EP 3 012 221 A1**
(43) Date of publication of application: **27.04.2016**
(21) Application number: 14382415.9
(22) Date of filing: 23.10.2014
(51) Int. Cl.: B82Y 5/00, B82Y 15/00, C12Q 1/68

(54) **Nucleic acid-induced aggregation of metal nanoparticles and uses thereof in methods for detecting nucleic acids**

(71) Applicant: Universitat Rovira I Virgili, 43003 Tarragona (ES); Institució Catalana de Recerca i Estudis Avançats, 08010 Barcelona (ES); Fundacio Centre Tecnologic de la Quimica de Catalunya, 43007 Tarragona (ES); University of Strathclyde, Glasgow, Strathclyde G1 1XB (GB); Medcom Tech, S.A., 28009 Madrid (ES)
(72) Inventor: Álvarez Puebla, Ramón Ángel, 43003 Tarragona (ES); Guerrini, Luca, 43003 Tarragona (ES); Sagalés Mañas, Juan, 28009 Madrid (ES); Graham, Duncan, Glasgow, Strathclyde UK-G1 1XB (GB)
(74) Representative: ABG Patentes, S.L.

(57) **Abstract**

The invention relates to an aggregate comprising metallic nanoparticles and nucleic acid molecules wherein each metallic nanoparticle is coated with a polycation. The invention also relates to the a method for obtaining the aggregate of the invention and to the use of said aggregate in methods for detecting the presence of a nucleic acid in a sample, in methods for detecting the presence of a given nucleotide at a predetermined position in a target nucleic acid, in methods for detecting the presence of a modified nucleotide at a predetermined position in a target nucleic acid, methods for detecting the presence of a conjugate between a double stranded nucleic acid and a chemical in a sample comprising double stranded nucleic acid molecules, in methods for determining the content of modified nucleotides in a target nucleic acid and in method for determining the content of modified nucleotides in a target nucleic acid.

SEQUENCE LISTING

<110>  UNIVERSITAT ROVIRA I VIRGILI
       INSTITUCIÓ CATALANA DE RECERCA I ESTUDIS AVANÇATS
       FUNDACIÓ CENTRE TECNOLÒGIC DE LA QUÍMICA DE CATALUNYA
       UNIVERSITY OF STRATHCLYDE
       MEDCOM TECH, S.A

<120>  NUCLEIC ACIDS-INDUCED AGGREGATION OF METAL NANOPARTICLES AND USES
        THEREOF IN METHODS FOR DETECTING NUCLEIC ACIDS

<130>  P11219EP00

<160>  14

<170>  PatentIn version 3.5

<210>  1
<211>  21
<212>  DNA
<213>  Artificial Sequence

<220>
<223>  ss1

<400>  1
catcgcaggt acctgtaaga g                                                21


<210>  2
<211>  21
<212>  DNA
<213>  Artificial Sequence

<220>
<223>  ss2

<400>  2
catcgcaggt acctgtaaga a                                                21


<210>  3
<211>  21
<212>  DNA
<213>  Artificial Sequence

<220>
<223>  ss3

<400>  3
aatcgcaggt acctgtaaga g                                                21


<210>  4
<211>  21
<212>  DNA
<213>  Artificial Sequence

<220>
<223>  ss4

<400>  4
catcgcaggt aactgtaaga g                                                21

<210>  5
<211>  21
<212>  DNA
<213>  Artificial Sequence

<220>
<223>  ss5

<400>  5
catcgcaggt tcctgtatga g                                                21


<210>  6
<211>  21
<212>  DNA
<213>  Artificial Sequence

<220>
<223>  ssmc



<220>
<221>  modified_base
<222>  (1)..(1)
<223>  m5c

<220>
<221>  modified_base
<222>  (4)..(4)
<223>  m5c

<220>
<221>  modified_base
<222>  (6)..(6)
<223>  m5c

<220>
<221>  modified_base
<222>  (12)..(12)
<223>  m5c

<220>
<221>  modified_base
<222>  (13)..(13)
<223>  m5c

<400>  6
catcgcaggt acctgtaaga g                                                21


<210>  7
<211>  21
<212>  DNA
<213>  Artificial Sequence

<220>
<223>  ssma


<220>
<221>  modified_base

<222>  (2)..(2)
<223>  m6a

<220>
<221>  modified_base
<222>  (7)..(7)
<223>  m6a

<220>
<221>  modified_base
<222>  (11)..(11)
<223>  m6a

<220>
<221>  modified_base
<222>  (17)..(17)
<223>  m6a

<220>
<221>  modified_base
<222>  (18)..(18)
<223>  m6a

<220>
<221>  modified_base
<222>  (20)..(20)
<223>  m6a

<400>  7
catcgcaggt acctgtaaga g                                                21


<210>  8
<211>  21
<212>  DNA
<213>  Artificial Sequence

<220>
<223>  ssc

<400>  8
gtagcgtcca tggacattct c                                                21


<210>  9
<211>  21
<212>  DNA
<213>  Artificial Sequence

<220>
<223>  pA

<400>  9
aaaaaaaaaa aaaaaaaaaa a                                                21


<210>  10
<211>  21
<212>  DNA
<213>  Artificial Sequence

<220>
<223>  pC

<400>  10
cccccccccc cccccccccc c                                                21

<210>  11
<211>  21
<212>  DNA
<213>  Artificial Sequence

<220>
<223>  pT

<400>  11
tttttttttt tttttttttt t                                                21

<210>  12
<211>  21
<212>  DNA
<213>  Artificial Sequence

<220>
<223>  pG

<400>  12
gggggggggg gggggggggg g                                                21

<210>  13
<211>  22
<212>  DNA
<213>  Artificial Sequence

<220>
<223>  ssCG

<400>  13
ccgcgccgcg cgcgcggcgc gg                                               22

<210>  14
<211>  22
<212>  DNA
<213>  Artificial Sequence

<220>
<223>  ssAT

<400>  14
aatataatat atatattata tt                                               22

## Description

### FIELD OF THE INVENTION

The invention relates to the field of spectroscopy and, more in particular, to the compositions and methods for detecting the presence of a nucleic acid in a sample, for detecting the presence of a given nucleotide at a predetermined position in a target nucleic acid, for detecting the presence of a modified nucleotides in a target nucleic acid and for detecting the presence of a conjugate between a double stranded nucleic acid and a chemical in a sample using Surface enhanced Raman scattering (SERS) spectroscopy.

### BACKGROUND OF THE INVENTION

Since the discovery of DNA structure, many methods had been developed for its evaluation. Nowadays, DNA analysis relies in restriction digestion, electrophoresis, polymerase chain reaction (PCR) for fragmentation, classification and amplification, respectively, and identification through *in situ* fluorescence or southern hybridization or similar. These methods are costly both in money and time and lose or not reveal information such the DNA methylation. The advent of nanophotonics and specially plasmonics offers a unique opportunity for the development of direct, fast and ultrasensitive methods for DNA analysis. Traditionally, this detection has relied almost exclusively on polymerase chain reaction (PCR) and DNA microarray techniques However, these methods require extrinsic labels (e.g., fluorophores or radiolabels) for detection of probe-target hybridization, which ultimately increases the cost and complexity of the detection assay. Further, DNA methylation information is erased by standard molecular biology techniques, such as cloning in bacteria and PCR, and it is not revealed by hybridization as the methyl group is located in the major groove of DNA rather than at the hydrogen bonds. Therefore, methylation dependent pretreatments of DNA has been developed to reveal the presence or absence of the methyl group at cytosine residues further increasing the time, complexity and cost of the DNA analysis. Thus, new genotyping sensitive and accurate methods with high throughput screening capabilities and affordable cost are urgently needed for gaining full access to the abundant genetic variation of organisms.

To address this issue, label-free techniques such as surface plasmon resonance (SPR) have been investigated Unfortunately, these methods detect only changes in mass and do not provide a chemical-specific readout. In the broad field of plasmonics, surface-enhanced Raman scattering (SERS) spectroscopy has arisen as a powerful analytical tool in the detection and structural characterization of biomolecules. The large majority of SERS DNA detection strategies emulates southern blot substituting the fluororescent or radiolabels with a SERS encoded particle. However, labeling of the DNA strands requires complex chemistry and costly chemicals and does not provide any chemical-specific information.

A second approach is based on the direct detection of the distinctive SERS signal from DNA strands directly adsorbed onto the nanostructured surface. This label-free strategy showed outstanding analytical potential, both in terms of sensitivity, down single-molecule detection and of selectivity, as demonstrated, for instance, by the straightforward identification of single-base mismatches, post-translational modifications dehydration-induced structural modifications, hybridization events in DNA. However, as performed to date, SERS analysis of double stranded DNA (dsDNA) in solution is largely hindered by the negative nature of the sugar and the phosphate backbone which inhibits the direct contact of the nucleotide chain with the nanostructured metallic surface (negative at the physiological pH). As a result, dsDNA SERS spectra usually suffer from inherent poor spectral reproducibility and limited sensitivity. In this regard, different approaches had been applied to improve the effective adsorption of dsDNA to the plasmonic surfaces. Thiolation of the DNA was carried out to promote the covalent binding to metal but this approach still requires a non-trivial modification of the strands and severely limits the sensitivity to only the first few nucleotides closer to the plasmonic substrate. Alternatively, positively charged aggregating agents have been employed to simultaneously neutralize the negatively charged phosphate backbone to promote the adsorption of the DNA/agent complex onto the metallic surface, and induce nanoparticle aggregation. However, the use of aggregating agents yield poor or null control over both the overall nanoparticle assembly and the final position of the DNA within the aggregate, markedly contributing to signal irreproducibility, and may induce a significant restructuring of the DNA duplex upon coordination with the positively charged molecules. Recently, highly concentrated silver colloids were used to physically entrap dsDNA within interparticle volume thus avoiding the need of external aggregating agents. However, such strategy proved to be effective only for relatively high dsDNA concentration (1 mg/mL).

Therefore, there is a need in the state of the art to provide systems based in the functionalization of aggregates of nanoparticles with label-free nucleic acids for nucleic acid detection based on detection of SESR spectra with high sensitivity and selectivity. acid that are more stable than those that are known today, as well as to develop a method which allows detecting alterations in a sequence of a nucleic acid of interest by means of using said nanoparticles.

### BRIEF DESCRIPTION OF THE INVENTION

The authors of the present invention have observed that positively charged metal nanoparticles coated with a polycation are capable of acting as stabilizing ligand in the presence of nucleic acids. These nanoparticles suffer aggregation in the presence of nucleic acid molecules by means of electrostatic interaction between the negative charges in the nucleic acid molecules and the positive charges of the polycation in the coats of said nanoparticles. These aggregates are useful for the identification of a nucleic acid in a sample since the presence of nucleic acid in said sample leads to increasing the SERS spectrum of said sample. Moreover, the aggregates are useful for detecting specific positions within a nucleic acid molecule, for detecting modified nucleotides and for detecting the presence of a conjugate between a nucleic acid and a conjugate based on the detection of the SERS of the sample.

Therefore, in a first aspect, the invention relates to an aggregate comprising metallic nanoparticles and nucleic acid molecules wherein each metallic nanoparticle is coated with a polycation and wherein said aggregate is formed by electrostatic interactions between the negative charges in the nucleic acid molecules and the positive charges of the polycation in the coats of said metallic nanoparticles, wherein said aggregate is not an aggregate of spermine-coated silver nanoparticles containing a single-stranded DNA modified with 5-FAM or Cy5 or a double stranded DNA modified with 5-FAM or Cy5.

In further aspects, the invention relates to methods as defined in the claims.

### BRIEF DESCRIPTION OF THE FIGURES

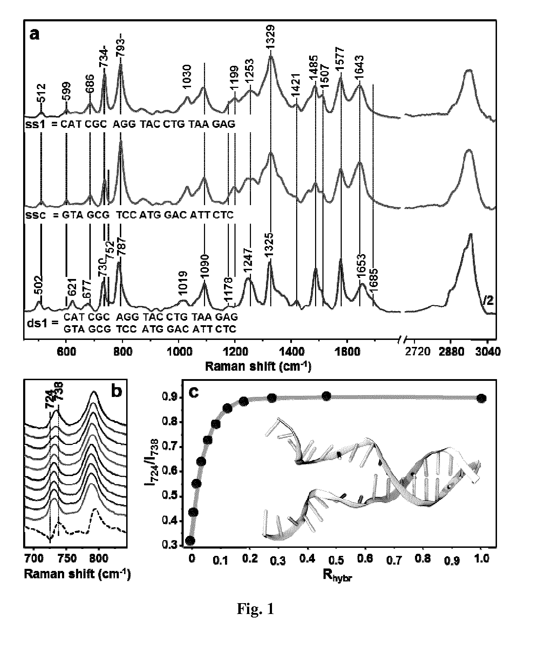

**Figure 1****.** *DNA hybridization: differentiation between single and double*-*stranded DNA sequences.* **(a)** SERS spectra of single stranded ss1 and ssc (5x10⁻⁷ M), and double stranded ds1 (5x10⁻⁸ M) on positively-charged silver nanoparticle colloids ([NP] *ca.* 0.3 nM). Difference spectra ds1-ss 1 and ds1-ssc are also reported. The spectra were normalized to the peak height of the phosphate band at 1090 cm⁻¹. **(b)** Detail of the 680-890 cm⁻¹ spectral region for the SERS spectra of ssc+ds1 mixtures at different molar ratio R_{hybr}= [ds1]/([ds1]+[ssc]) (from the top to the bottom: 0.011, 0.024, 0.041, 0.063, 0.091, 0.130, 0.189, 0.286, 0.474 and 1). The ds1 concentration was progressively increased from zero to 5x10⁻⁸ M (final concentration in the colloidal sample) while the ssc concentration was simultaneously decreased from 5x10⁻⁷ M to zero (final concentration in the colloidal sample). The dotted line is the difference spectrum obtained by subtracting the SERS spectra of ds1 (5x10⁻⁸ M) to the spectrum of ssc (5x10⁻⁷ M). **(c)** Ratiometric peak intensities I₇₂₄/I₇₃₈ vs. R_{hybr} = [ds1]/([ds1]+[ssc]) molar ratios (linear and logarithmic scale, respectively).

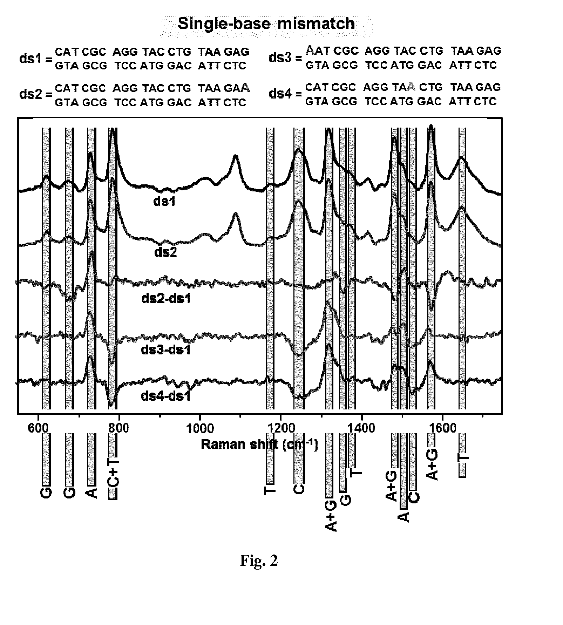

**Figure 2****.** *Endogenous DNA modifications: Single-base Mismatch* **(a)** SERS spectra of ds1 and ds2; and digitally subtracted SERS spectra (ds2-ds1), (ds3-ds1) and (ds4-ds1). 5x10⁻⁸ M is the final concentration of the duplexes in the positively-charged silver nanoparticle colloids ([NP] ca. 0.3 nM). For the sake of clarity, the digitally subtracted spectra were multiplied by 3 (for ds2-ds1) and 4 (for ds3-ds1 and ds4-ds1). Frequency positions of characteristic nucleotide bands are also highlighted.

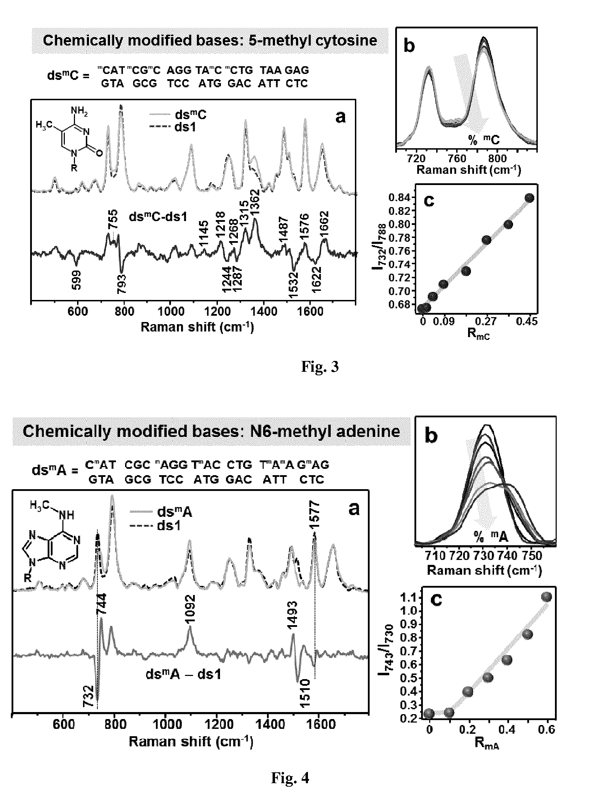

**Figure 3****.** *Endogenous DNA modifications: Chemically-modified bases, 5-methyl Cytosine* **(a)** Molecular structure of 5-methyl Cytosine (^{m}C). SERS spectra of ds1 and ds^{m}C, and the difference spectrum ds^{m}C-ds1. For the sake of clarity, the digitally subtracted spectra were multiplied by a factor of 2. **(b)** Detail of the 700-840 cm⁻¹ region for the SERS spectra of ds1+ds^{m}C at different cytosine base ratios R_{mC} = [^{m}C]/([C]+[^{m}C]) (from the top to the bottom, R_{mC} = 0; 0.045; 0.091; 0.182; 0.273; 0.364; and 0.455). The overall ds concentration was kept constant at 5x10⁻⁸ M. [NP] ca. 0.3 nM. (c) Ratiometric peak intensities I₇₃₂/I₇₈₈, vs. the molar ratio R_{mC} = [^{m}C]/([C]+[^{m}C]).
**Figure 4****.** *Endogenous DNA modifications: Chemically-modified bases, N5-methyl Adenine (**a**)* Molecular structure of N6-methyl Adenine (^{m}A). SERS spectra of ds1 and ds^{m}A, and the difference spectrum ds^{m}A-ds1. For the sake of clarity, the digitally subtracted spectra were multiplied by 2. (b) Detail of the 700-760 cm⁻¹ region for the SERS spectra of ds1+ ds^{m}A at different adenine base ratios R_{mA} = [^{m}A]/([A]+[^{m}A]) (from the top to the bottom R_{mA} = 0; 0.1; 0.2; 0.3; 0.4; 0.5 and 0.6). The overall ds concentration was kept constant at 630 ng/mL. (c) Ratiometric peak intensities I₇₄₃/I₇₃₀, vs. molar ratio R_{mA}.

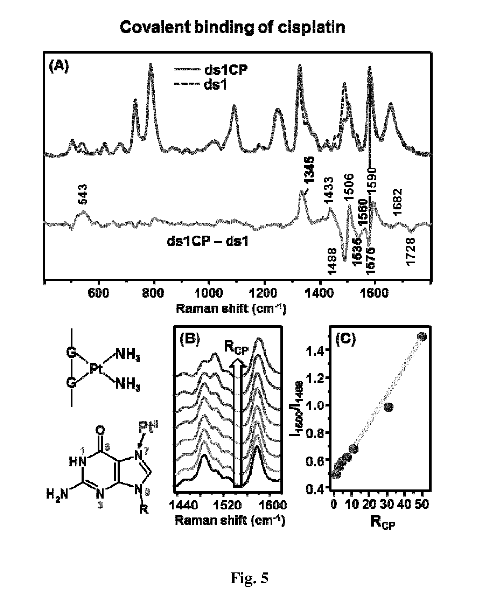

**Figure 5****.** *Exogenous DNA modifications: Formation of DNA adducts by cisplatin.* **(a)** SERS spectra of ds1 and ds1+CP mixture (R_{CP} = [CP]/[ds1] = 50), and the corresponding difference spectrum ds1CP-ds1. (b) Detail of the 1380-1620 cm⁻¹ spectral region for the SERS spectra of ds1+CP mixtures at different molar ratios (from the top to the bottom, R_{CP} = 50, 30, 10, 7, 4, 2 and 0). The final ds1 concentration was kept fixed through the entire study at 5x10⁻⁸ M whereas the CP concentration was modified accordingly ([NP] ca. 0.3 nM). (c) Ratiometric peak intensities I₁₅₉₀/I₁₄₈₈ vs. R_{CP}. Inset: schematic representation of the CP 1,2-intrastrand crosslink between neighboring G bases *via* binding to the N7 atom.

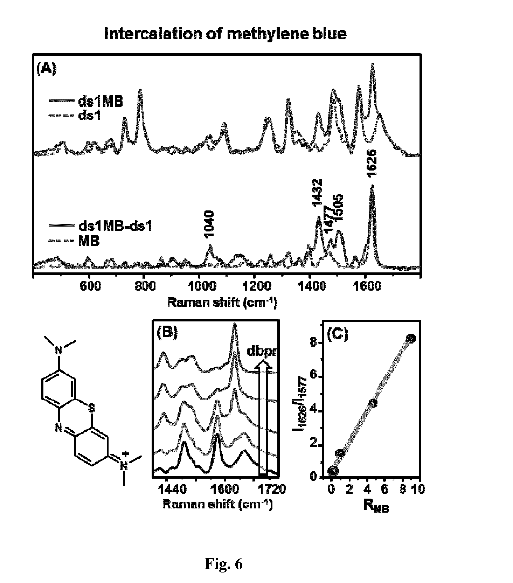

**Figure 6****.** *Exogenous DNA modifications: Intercalation of the organic dye methylene blue into the dsDNA.* **(a)** SERS spectra of ds1 and the equimolar ds1+MB mixture, and the calculated difference spectrum ds1MB-ds1. The SERS spectrum of MB (5x10⁻⁷ M) directly adsorbed onto positively-charged silver nanoparticles is also included. **(b)** Details of the 1400-1800 cm⁻¹ spectral region of the SERS spectra of ds1+MB mixtures at different molar ratios (from the top to the bottom, R_{MB} = 9.0, 4.5, 0.9, 0.009 and 0). The concentration of ds1 was kept constant at 5x10⁻⁸ M whereas the MB amount was modified accordingly ([NP] ca. 0.3 nM). (C) Intensity ratio between the MB band at 1625 cm⁻¹ and the ds1 band at 1577 cm⁻¹ (I₁₆₂₅/I₁₅₇₇) vs. R_{MB}. *Inset:* molecular structure of MB.

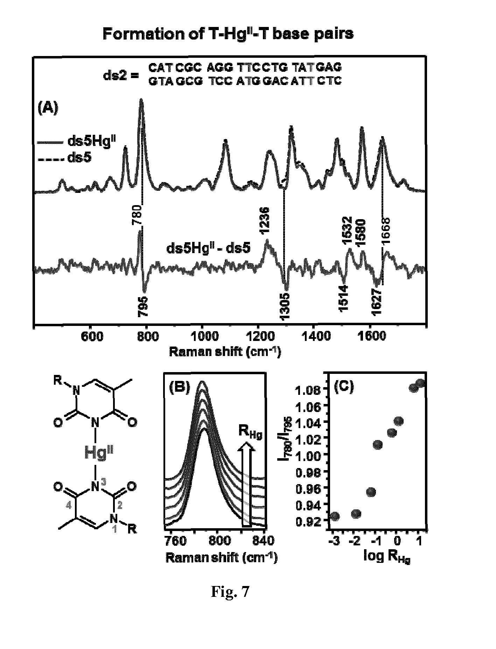

**Figure 7****.** *Exogenous DNA modifications: DNA-metal ion coordination: formation of T-Hg^{II}-T base pairs.* **(a)** SERS spectra of ds5 and the equimolar ds5+Hg^{II} mixture (R_{Hg} = [Hg^{II}]/[ds5] = 1), and the corresponding difference spectrum ds5Hg^{II}-ds5. **(b)** Detail of the 750-840 cm⁻¹ spectral region for the SERS spectra of ds5+Hg^{II} mixtures at different molar ratios R_{Hg} (from the top to the bottom: 0:1; 0.05:1, 0.1:1, 0.5:1, 1:1 and 5:1, corresponding to metal-to-T:T base pair ratios 0; 0.025; 0.05; 0.25; 0.5 and 2.5, respectively). The concentration of ds2 was kept constant at 5x10⁻⁸ M whereas the Hg^{II} amount was modified accordingly ([NP] ca. 0.3 nM). (c) Ratiometric peak intensities I₇₈₀/I₇₉₅, vs. R_{Hg} molar ratio in logarithmic scale. Inset: outline of the Hg^{II} insertion into the T:T mismatch via binding of the N3 atoms.

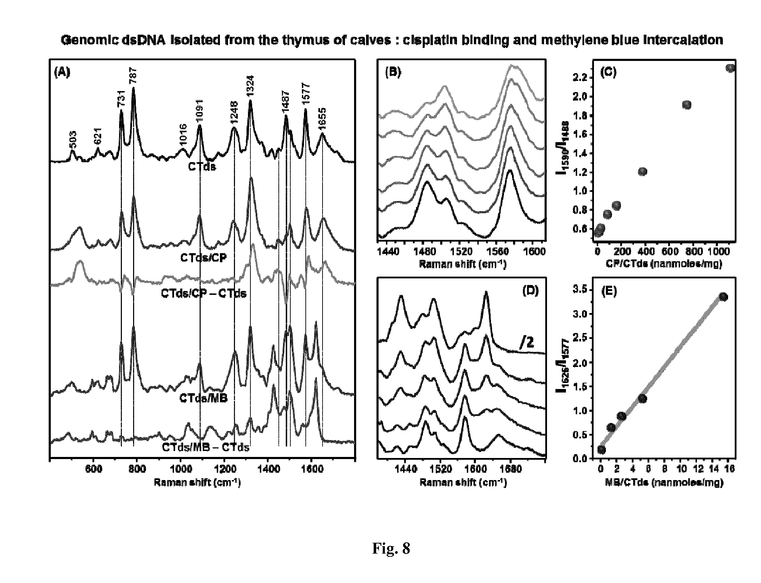

**Figure 8****. Cisplatin and methylene blue complexation of genomic CTds. (A)** SERS spectra of CTds and the mixtures CTds+CP and CTds+MB (1125 nanomoles of CP per mg of DNA and 5.15 nanomoles of MB per mg of DNA, respectively). The corresponding difference spectra (CTds/CP - CTds) and (CTds/MB - CTds) are also illustrated. **(B)** Detail of the 1430-1620 cm⁻¹ spectral region for the SERS spectra of CTds+CP mixtures at different nanomoles of CP/mg of DNA ratios (from the top to the bottom: 750; 375; 163; 81; 41 and 0). (C) Ratiometric peak intensities I₁₅₉₀/I₁₄₈₈ vs. CP/CTds ratio (nanomoles/mg). (D) Details of the 1380-1740 cm⁻¹ spectral region of the SERS spectra of CTds+MB mixtures at different nanomoles of MB/mg of DNA ratios (from the top to the bottom: 15.4; 5.1; 2.6; 1.3 and 0). (E) Ratiometric peak intensities I₁₆₂₆/I₁₅₇₇ vs. MB/CTds ratio (nmol/mg). The concentration of CTds was kept constant at 7.8 µg/mL for the whole study whereas CP and MB amounts were modified accordingly.

### DETAILED DESCRIPTION OF THE INVENTION

The inventors of the present invention have discovered that, surprisingly, metal nanoparticles coated with a polycation, acting as stabilizing ligand, undergo nanoparticle aggregation in the presence of nucleic acid molecules. Due to the electromagnetic properties of the nanoparticles which form said aggregates, the presence and properties of the nucleic acid molecules forming the aggregates are detected by SERS.

Based on these findings, the inventors have developed the compositions and methods of the present invention in their different embodiments that will be described now in detail.

### Aggregates of the invention

In a first aspect, the invention relates to an aggregate comprising metallic nanoparticles and nucleic acid molecules wherein each metallic nanoparticle is coated with a polycation and wherein said aggregate is formed by electrostatic interactions between the negative charges in the nucleic acid molecules and the positive charges of the polycation in the coats of said metallic nanoparticles, wherein said aggregate is not an aggregate of spermine-coated silver nanoparticles containing a single-stranded DNA modified with 5-FAM or Cy5 or a double stranded DNA modified with 5-FAM or Cy5.

The term "aggregate" as used herein, refers to an entity formed by metallic nanoparticles and nucleic acid molecules which are associated with one other as a result of the interaction between them due to the electrostatic interactions between the negative charge in the nucleic acid molecules and the positive charges of the polycation in the coats of said metallic nanoparticles. As a result of said aggregation, aggregates formed by dimers, trimmers, and aggregates of higher order will be generated.

The term "nanoparticle" is used to designate colloidal systems of the spherical type, rod type, polyhedron type, etc., or similar shapes, having a size less than 1 micrometer (µm), which are individually found or are found forming organized structures (dimers, trimers, tetrahedrons, etc.), dispersed in a fluid (aqueous solution). In a particular embodiment, the nanoparticles suitable for putting the invention into practice have a size less than 1 µm, generally comprised between 1 and 999 nanometers (nm), typically between 5 and 500 nm, preferably between about 10 and 150 nm. In a particular embodiment, the nanoparticles of the invention typically have a mean particle diameter ranging from 2 to 100 nm, preferably from 15 to 80 nm. The mean particle diameter is the maximum mean particle dimension, with the understanding that the particles are not necessarily spherical. The shape of said nanoparticles can widely vary; advantageously, said nanoparticles will adopt any optically efficient shape such as spheres, rods, stars, cubes, polyhedrons or any other variant as well as complex associations of several particles; in a particular embodiment, the shape of the nanoparticles for putting the invention into practice is spherical or substantially spherical. The shape can be suitably evaluated by conventional light or by means of electron microscopy techniques.

The core of the nanoparticles comprises one or more particles of any suitable material known in the art such as, for example, any metals and doped semiconductors that can sustain Raman signal amplification are suitable for use in the present invention.

The core of said nanoparticles can be prepared with a material capable of generating high electric or electromagnetic fields at the particle surface by means of the interaction thereof with a light beam, such as materials which generate surface plasmon resonances or "whispering gallery modes" (Mie Resonances) excited by means of monochromatic light beams, for example, lasers, LEDs, OLEDs, lamps with filters, etc. Non-limiting illustrative examples of said material include: plasmonic materials comprising metals such as gold, silver copper, aluminum, rhodium, ruthenium, indium, alkaline metals, alkaline-earth metals, the alloys of these metals, the alloys of these metals with other metals, etc.); as well as semiconductors in which plasmons are generated by inducing vacancies in the crystalline structure, for example, CuSe, CuSe or CuTe; and materials with a high refractive index capable of sustaining Mie resonances, such as silicon, etc. The plasmonic materials provide the electromagnetic field necessary for enhancing the SERS signal of the Raman molecule. In a particular embodiment, the metal is silver or gold or a combination thereof. Thus, in a particular embodiment, the aggregate of the invention comprises gold nanoparticles. In another particular embodiment, the aggregate of the invention comprises silver nanoparticles. In another particular embodiment, the aggregate of the invention comprises bimetallic silver-gold nanoparticles.

The aggregate according to the present invention also comprises nucleic acid molecules. The term "nucleic acid" as used herein, refers to polymers formed by the repetition of nucleotides bound by means of phosphodiester bonds. Generally, nucleic acids store the genetic information of living organisms. There are two types of nucleic acids: double and single stranded DNA molecules (deoxyribonucleic acid) and double and single stranded RNA molecules (ribonucleic acid). This term includes modified nucleic acids and conjugated nucleic acids. The term "nucleic acid" also refers to molecules formed by non-conventional nucleotides bound as well as variants thereof, including modifications in the purine or pyrimidine residues and modifications in the ribose or deoxyribose residues designed for increasing biological stability of the oligonucleotide or for stabilizing the physical stability of the hairpin-shaped structure. Examples of modified nucleotides that can be used in the present invention include, but are not limited to, nucleotides having at position 2' of the sugar a substituent selected from the fluoro, hydroxyl, amino, azido, alkyl, alkoxy, alkoxyalkyl, methyl, ethyl, propyl, butyl group or a functionalized alkyl group such as ethylamino, propylamino and butylamino. Alternatively, the alkoxy group is methoxy, ethoxy, propoxy or a functionalized alkoxy group according to the formula -O(CH₂)q-R, where q is 2 to 4 and R is an amino, methoxy or ethoxy group. Suitable alkoxyalkyl groups are methoxyethyl and ethoxyethyl. Oligonucleotides in which different nucleotides contain different modifications at position 2' are also part of the invention. Alternatively or additionally, the oligonucleotides of the invention can contain modified bonds such as phosphodiester-, phosphotriester-, phosphorothioate-, phosphorodithioate-, phosphoroselenoate-, phosphorodiselenoate-, phosphoroanilothioate-, phosphoramidate-, methylphosphonate-, boranephosphonate-type bonds as well as combinations thereof, or they are peptide nucleic acids (PNAs), in which the different nucleotides are bound by amide bonds. The chemical synthesis of nucleic acids is a well-known technique and it can be performed, for example, by means of using an automatic DNA synthesizer (such as commercially available Bioautomation synthesizers, for example).

In a particular embodiment, said nucleic acid forming the aggregate of the invention is selected from the group consisting of RNA, DNA, a double stranded nucleic acid, a single stranded nucleic acid, methylated DNA, a coordination complex of a nucleic acid and a metal, a coordination complex of a nucleic acid and a compound containing a metal and a complex of a nucleic acid and an intercalating organic dye.

The term "methylated DNA", as used herein, refers to the presence of methyl group in one or more nucleotides forming part of the nucleic acid molecule.

The term "coordination complex", as used herein, refers to a central atom or ion, which is usually metallic and is called the coordination centre and a surrounding bound molecules or ion, i.e. nucleic acids that are known as ligand or complexing agents. Ligands are generally bound by a coordinate covalent bond (donating electrons from a ion electron pair into an empty metal orbital), and are said to be coordinated to the atom. In a particular embodiment, said metal is Hg(II).

The present invention also contemplates a coordination complex of a compound containing a metal and a nucleic acid. In a particular embodiment, said compound containing a metal is cisplatin. Cisplatin is formed by a platinum atom with four ligands, namely, two chloride ligands and two amine ligands.

The term "intercalating organic dye", as used herein, refers to an organic molecule which binds to a nucleic acid molecule. The binding interaction between said molecule and the nucleic acid molecule leads to a significant change in the nucleic acid structure and may have influence on its functions. Illustrative and no limitative examples of intercalating organic dye which can be used according to the present invention are, phenonthiazinium dyes such as methylene blue, cyanine dyes such as cyan2, and phenathridium ions such as ethidium bromide. In a preferred embodiment, said intercalating organic dye is methylene blue.

The term "polycation", as used herein, refers to a cation having more than one positive charge. Illustrative and non-limitative examples of polycations which can be used according to the present invention are ethylene diamine, 1,3-diaminopropane, hexamethylenediamine, putrescine, cadaverine, spermine, spermidine and putrescine. In a particular embodiment, said polycation which coats the aggregate of the invention is spermine, spermidine or putrescine. In a preferred embodiment, said polycation is spermine.

As has been mentioned above, each metallic nanoparticle is coated with a polycation. The coating provides the core (which is surrounded/coated by the coating) with mechanical and chemical stability, prevents the core from exterior reactions, and renders the core amenable to use in many solvents without disrupting the SERS response. This core and coating structure is well-known for the skilled person in the art. Methods for preparing a coating comprising silica are also well-known to those of skill in the art.

The thickness of the coating may vary. By illustrative, and without wishing to be limiting in any manner, the thickness of the coating may be applied in a controlled manner. The thickness of the coating, once complete, may be about 1 nm and 100 nm, or any value there between; for example, the polycation coating may be about 1, 5, 10, 15, 20, 25, 30, 35, 40, 45, 50, 55, 60, 65, 70, 75, 80, 85, 90, 95, or 100 nm thick, or any value between. In a specific, non-limiting example, the thickness of the coating may be about 10 to 30 nm.

The aggregate of the invention is formed by electrostatic interactions between the negative charges in the nucleic acid molecules and the positive charges of the polycation coats of said metallic nanoparticles. The term "electrostatic interaction" as used herein, refers to the force between charged particles caused by their electric charges. Said electrostatic force is considered as "electrostatic force of attraction" when it occurs between two polar (charged) opposites, namely, positive and negative charges (i.e. the force occurring between a cation and anion in an ionic molecule). By the contrary, the electrostatic force of repulsion is understood as the tendency of similarly- charged particles to separate from each other. Due to said electrostatic interaction between the negative charges in the nucleic acid molecules and the positive charges of the polycation coats of said metallic nanoparticles, the acid nucleic molecule is adsorbed onto the metallic nanoparticle

The polycation coat provides a metallic nanoparticle having a positive surface electrostatic charge. The term "surface electrostatic charge", as used herein, refers to the electrostatic charge or the nanoparticle that can be measured by the zeta potential of the nanoparticle. The term "zeta potential" refers to the electric potential in the interfacial double layer (DL) at the location of the slipping plane versus a point in the bulk fluid away from the interface. In other words, zeta potential is the potential difference between the dispersion medium and the stationary layer of fluid attached to the dispersed particle. The person skilled in the art knows how to calculate the zeta potential of a nanoparticle based on an experimentally-determined electrophoretic mobility or dynamic electrophoretic mobility.

The person skilled in the art understands that the conditions under which said aggregate of the invention is formed include an appropriate concentration of desired metallic nanoparticles and an appropriate concentration of the nucleic acid; thus an appropriate ratio between metallic nanoparticles/nucleic acid. As the person skilled in the art knows, the ratio may be expressed in terms of molarity, i.e. molar ratio The term "molar ratio" as used herein refers to the ratio between the amounts in moles of any two compounds (i.e. nucleic acids and nanoparticles) involved a reaction or interaction. In a preferred embodiment, said molar ratio between metallic nanoparticles/nucleic acid is at least from about 1:1000 to about 1000:1, at least from about 1:900 to 900:1, at least from about 1:800 to 800:1, at least from about 1:700 to 700:1, at least from about 1:600 to 600:1, at least from about 1:500 to 500:1, at least from about 1:400 to 400:1, at least from about 1:300 to 300:1, at least from about 1:200 to 200:1, at least from about 1:100 to 100:1, at least from about 1:90 to 90:1, at least from about 1:80 to 80:1, at least from about 1:70 to 70:1, at least from about 1:60 to 60:1, at least from about 1:50 to 50:1, at least from about 1:40 to 40:1, at least from about 1:30 to 30:1, at least from about 1:20 to 20:1, at least from about 1:10 to 10:1, at least from about 1:5 to 5:1, at least from about 1:2 to 2:1. In another preferred embodiment said molar ratio is 1:100. In another preferred embodiment said molar ratio is 1:103 or more. In a more preferred embodiment said molar ratio is 1:1000. In another preferred embodiment said molar ratio is 1:166. In another preferred embodiment said molar ratio is 1:3. In another preferred embodiment said molar ratio is 1:833.

The ratio may also be expressed in terms of weight (i.e. grams of nanoparticles/ grams of nucleic acid), in terms of mass concentration (weight/volume). In a preferred embodiment, the ratio is expressed in terms of weight (i.e. nucleic acid)/molar concentration (nanoparticles). In a preferred embodiment the ratio is at least from about 0.1 to 1, at least from about 1 to 5, at least from about 5 to 10, at least from about 10 to 20, at least from about 20 to 30, at least from about 30 to 40, at least from about 40 to 50, at least from about 50 to 60, at least from about 60 to 70, at least from about 70 to 80, at least from about 80 to 90, at least from about 90 to 100, at least from about 100 to 110, at least from about 110 to 120, , at least from about 120 to 130, at least from about 130 to 140, , at least from about 140 to 150, at least from about 150 to 160, at least from about 160 to 170, at least from about 170 to 180, at least from about 180 to 190, at least from about 190 to 200. In a more preferred embodiment, said ratio is 1 µg of nucleic acid/0.3 nM of nanoparticles. In another preferred embodiment, said ratio is 3 µg of nucleic acid/0.3 nM of nanoparticles. In another preferred embodiment, said ratio is 3 µg of nucleic acid/0.3 nM of nanoparticles.

### Method for obtaining the aggregates of the invention

In a second aspect, the invention relates to a method for obtaining the aggregate of the invention, hereinafter, "the first method of the invention", comprising the steps of:
(i) obtaining a population of metallic nanoparticles by contacting a salt of a metal and a hydrochloride of a polycation in the presence of a reducing agent under conditions adequate for the formation of the metallic nanoparticles coated with said polycation; and
(ii) contacting the nanoparticles obtained in step (i) with a nucleic acid under conditions adequate for the formation of an aggregate formed by electrostatic interaction between a negatively charged nucleic acid and the positive charges of the polycation in the coat of said metallic nanoparticles.

The terms "aggregate", "metallic nanoparticle", "polycation", "nucleic acid", "electrostatic interaction" as well as their particular embodiments thereof are defined in the first aspect of the invention and equally apply to the second aspect of the invention.

The first step of the first method of the invention comprises obtaining a population of metallic nanoparticles by contacting a salt of a metal and a hydrochloride of a polycation in the presence of a reducing agent under conditions adequate for the formation of the metallic nanoparticles coated with said polycation The term "population of metallic nanoparticles", as used herein, refers to a set formed by at least 2 metallic nanoparticles, at least 3 metallic nanoparticles, at least 4 metallic nanoparticles, at least 5 metallic nanoparticles, at least 10 metallic nanoparticles, at least 20 metallic nanoparticles, at least 30 metallic nanoparticles, at least 40 metallic nanoparticles, at least 50 metallic nanoparticles, at least 100 metallic nanoparticles or more.

The obtention of the metal nanoparticles according to the first step of said method can be carried out using any conventional methodology with the proviso that said process is carried out in presence of a hydrochloride of a polycation. The general method for obtaining metal nanoparticles is based on the preparation of an aqueous solution comprising one or more metal salts to which there is added a reducing agent capable of reducing the metal salt cation to the metal state.

In a particular embodiment of the invention, the first step of the first method of the invention relates to obtaining silver, gold or bimetallic silver-gold nanoparticles. In a preferred embodiment of the invention, said metallic nanoparticles to be obtained are silver nanoparticles. In a preferred embodiment of the invention, the metal salts used for obtaining said nanoparticles comprise silver salts, more preferably AgNO₃.

If desired, the method for obtaining metal nanoparticles can be carried out by means of the method of chemical reduction of Ag⁺ using any suitable reducing agent. Illustrative examples of reducing agents are mentioned below.

The term "hydrochloride", as used herein, refers to a salt resulting or regarded as resulting, from the reaction of hydrochloric acid with an organic base (such NH₃ groups). Hydrochloride polycations which can be used according to the first step of the present method are well known in the state of the art. Illustrative examples of hydrochloride polycations which can be used in this context of the invention are any suitable hydrochloride polyamine such spermine hydrochloride, spermidine hydrochloride or putrescine hydrochloride. In a preferred embodiment, said hydrochloride polycation is spermine hydrochloride.

The term "obtaining a population of metallic nanoparticles by contacting a salt of a metal and a hydrochloride of a polycation in the presence of a reducing agent", as used herein, refers to incubation of said metallic salt from which metallic nanoparticles are formed, preferably, silver salt, and said polycation hydrochloride, preferably spermine hydrochloride, in the presence of a reducing agent under conditions adequate for the interaction between said metallic salt and said polycation hydrochloride, and under conditions which allow the reduction of the metal salt cation to the metal state; thereby leading the formation of a metallic nanoparticles coated by said hydrochloride of a polycation without disrupting the SERS response. The term "in the presence of a reducing agent" as used herein, refers to incubation of said metallic salt with the polycation under conditions adequate for the interaction between said metallic salt and said polycation hydrochloride wherein the solution in which said interaction is carried out contains said reducing agent, Said term also refers to incubation of said metallic salt with the polycation under conditions adequate for the interaction between said metallic salt and said polycation hydrochloride wherein the reduced agent is added after said interaction has occurs. The term "after said interaction has occurs" as used herein refers to suitable time that allows the interaction between said metallic salt and said polycation hydrochloride. Thus, reducing agent can be added to the solution containing said metallic salt and said polycation hydrochloride 10 sec, 30 sec, 1 min, 5 min, 10 min, 15 min, 20 min, 25 min, 30 min, 35 min, 40 min, 45 min, 50 min, 60 min or more after the metallic salt and the hydrochloride polycation are put in contact.

Said term also refers to incubation of a salt of a metal and a hydrochloride of a polycation under conditions adequate for the interaction between said metallic salt and said polycation hydrochloride wherein the reducing agent is added before said interaction occurs. In this event, due to the reduction of the metal salt cation to the metal state, metallic nanoparticles will be obtained before contacting them with said hydrochloride of a polycation. The term "before said interaction has occurs" as used herein refers to suitable time that allows the interaction between said metallic salt and said reducing agent giving rise to metallic nanoparticles. Thus, said hydrochloride of a polycation can be added to the solution containing said metallic salt and said reducing agent 10 sec, 30 sec, 1 min, 5 min, 10 min, 15 min, 20 min, 25 min, 30 min, 35 min, 40 min, 45 min, 50 min, 60 min or more after the metallic salt and the reducing agent are put in contact.

The person skilled in the art understands that the conditions under which said covered metallic nanoparticles covering is carried out include an appropriate concentration of desired metal salt for obtaining the metallic nanoparticles, an appropriate concentration of the polycation hydrochloride as well as appropriate conditions of the reducing agent, salt, pH, temperature and time of contacting. Starring is also preferred. The person skilled in the art also understands that the molar ratio of the metallic salt to the polycation can be turned in order to avoid uncontrolled aggregation of the nanoparticles. In a preferred embodiment of the invention, the ratio of the metallic salt concentration to the polycation concentration is from about 100:1 M to 1:1 mol/mol. In another preferred embodiment of the invention, the ratio of the metallic salt concentration to the polycation concentration is from about 90:1 mol/mol to about 10:1 mol/mol. In another preferred embodiment of the invention, the ratio of the metallic salt concentration to the polycation concentration is from about 80: 1 mol/mol to about 20:1 mol/mol. In another preferred embodiment of the invention, the ratio of the metallic salt concentration to the polycation concentration is from about 70:1 mol/mol to about 30:1 mol/mol. In another preferred embodiment of the invention, the ratio of the metallic salt concentration to the polycation concentration is from about 60:1 mol/mol to about 40:1 mol/mol. In another preferred embodiment of the invention, the ratio of the metallic salt concentration to the polycation concentration is of about 50:1 mol/mol.

The reducing agents capable of reducing the metal salt cation to the metal state are known by the person skilled in the art. Hydrides, citrates, alcohols, reducing polymers, hydrazine, hydroxylamine or their derivatives and mixtures thereof can be used as reducing agents. In a particular and preferred embodiment of the invention, said reducing agent is a borohydride.

In a preferred embodiment of the first method of the invention, is carried out as follows: an appropriate concentration of silver salt, (AgNO₃), i.e., about 973 µM (final concentration) is added to any suitable solvent, such water, preferably, Milli-Q water, followed by addition of any suitable polycation, preferably spermine tetrathydrocloride in an appropriate concentration i.e. 68 µM (final concentration) and an appropriate concentration of the reducing agent, preferably borohydre, i.e. 243 µM (final concentration) which is added under starring.

Suitable concentration of a desired metallic salt, preferably AgNO₃, include without limitation 0.1 mM, 0.5 mM, 1 mM, 2 mM, 3 mM, 4 mM, 5 mM, 6 mM, 7 mM, 8 mM, 9 mM, 10 mM, 0.1 M, 0.2 M, 0.3M, 0.4 M, 0.5 M or 1 M; suitable concentration of said hydrochloride of a polycation, preferably, spermine hydrochloride, include without limitation 0.001mM, 005 mM, 0.1 mM, 0.5 mM, 1 mM, 2 mM, 3 mM, 4 mM, 5 mM, 0.1 M, 0.2 M, 0.3M, 0.4 M, 0.5 M or 1 M; suitable concentration of said reducing agent, include without limitation, 0.001 mM, 0.005 mM, 0.01 mM, 0.05 mM, 0.1 mM, 0.5 mM, 1 mM, 2 mM, 3 mM, 4 mM, 5 mM, 10 mM, 0.1 M, 0.2 M, 0.3M, 0.4 M, 0.5 M or 1 M; pH values include values of 5, 5.5, 6, 6.5, 7, 7.5; suitable time of contacting include 5 min, 10 min, 15 min, 20 min, 25 min, 30 min, 35 min, 40 min, 45 min, 50 min, 60 min or more. Preferred purified water is desirable for carry out the contacting between said metallic nanoparticle and said polycation hydrochloride.

Once that said coated metal nanoparticles in solution are obtained, if desired, they are collected, preferably by means of filtration or centrifugation, and optionally purified. The purification of the nanoparticles can be carried out by means of processes of washing/centrifuging in a suitable solvent, preferably water, or by means of dialysis. The nanoparticles thus obtained can be detected by UV-Vis spectroscopy, transmission electron microscopy (TEM) or Raman spectroscopy. In a preferred embodiment, the nanoparticles of the invention are detected by means of TEM and/or UV-Vis spectroscopy at 390-400 nm. The concentration of said nanoparticles can be estimated by means of any technique suitable for that purpose. By way of illustration, an estimation of the concentration of the obtained nanoparticles can be determined by means of the Lambert-Beer law or by means of determining the molar extinction coefficient, which is a parameter that defines the radiation absorbed by a substance at a certain wavelength per molar concentration. The nanoparticles obtained according to the first step of the invention can be stored. In this case it is advisable to store said nanoparticles under conditions that avoid deterioration thereof. The nanoparticles can be stored at a temperature between about 4°C and 30°C. It is also recommendable using materials previously treated with any suitable polycation (such as polyethylenemimine) in order to prevent unspecific attachment of positive charged metallic nanoparticles to storage surfaces.

The second step of the first method of the invention comprises contacting the nanoparticles obtained in the first step of said method with a nucleic acid under conditions adequate for the formation of an aggregate formed by electrostatic interaction between a negatively charged nucleic acid and the positive charges of the polycation in the coats of said metallic nanoparticles. As the person skilled in the art will understand, in this context, the nucleic acid may be added to the suspension containing metal nanoparticles coated with a polycation or, alternatively, a suitable solution could be prepared from a suspension containing said metallic nanoparticles and a suitable solvent. The nucleic acid that is bound or adsorbed to the metallic nanoparticles coated with a polycation will be then added to said solution. Said conditions are suitable for the spontaneous electrostatic interaction between a negatively charged phosphate groups of the nucleic acid and the positive charges of the polycation in the coats of said metallic nanoparticles, thereby, forming an aggregate of nanoparticles as defined in the first aspect of the invention. Said conditions are those which do not modify the negative charge of the nucleic acid and which do not modify the positive charge of the polycation in the coats of the metallic nanoparticles. Since pH and ionic strength have marked effect on the electric charge of charged molecules, special careful is taken when choosing the suitable solution for carry out the second step of the first method of the invention. The conditions under which said covering is carried out include an appropriate concentration of metallic nanoparticles obtained in the first step, an appropriate concentration of a nucleic acid and appropriate conditions of salt, pH, temperature and time of contacting. The ratio of nanoparticles to the nucleic acid must be adjusted in order to avoid large formation of unstable aggregates (high nanoparticle-to-nucleic acid ratios) or limited formation of stable aggregates (low nanoparticle-to-nucleic acid ratios).

In a preferred embodiment, said ratio between metallic nanoparticles/nucleic acid refers to a molar ratio between said metallic nanoparticles/nucleic acid. In a preferred embodiment, said molar ratio is at least from about 1:1000 to about 1000:1, at least from about 1:900 to 900:1, at least from about 1:800 to 800:1, at least from about 1:700 to 700:1, at least from about 1:600 to 600:1, at least from about 1:500 to 500:1, at least from about 1:400 to 400:1, at least from about 1:300 to 300:1, at least from about 1:200 to 200:1, at least from about 1:100 to 100:1, at least from about 1:90 to 90:1, at least from about 1:80 to 80:1, at least from about 1:70 to 70:1, at least from about 1:60 to 60:1, at least from about 1:50 to 50:1, at least from about 1:40 to 40:1, at least from about 1:30 to 30:1, at least from about 1:20 to 20:1, at least from about 1:10 to 10:1, at least from about 1:5 to 5:1, at least from about 1:2 to 2:1. In another preferred embodiment said molar ratio is 1:100. In another preferred embodiment said molar ratio is 1:103 or more. In a more preferred embodiment said molar ratio is 1:1000. In another preferred embodiment said molar ratio is 1:166. In another preferred embodiment said molar ratio is 1:3. In another preferred embodiment said molar ratio is 1:833.

In another preferred embodiment, said ratio refers to the weight of said nucleic acid to molar concentration of said metallic nanoparticles. In this event, in a preferred embodiment the ratio is at least from about 0.1 to 1, at least from about 1 to 5, at least from about 5 to 10, at least from about 10 to 20, at least from about 20 to 30, at least from about 30 to 40, at least from about 40 to 50, at least from about 50 to 60, at least from about 60 to 70, at least from about 70 to 80, at least from about 80 to 90, at least from about 90 to 100, at least from about 100 to 110, at least from about 110 to 120, , at least from about 120 to 130, at least from about 130 to 140, , at least from about 140 to 150, at least from about 150 to 160, at least from about 160 to 170, at least from about 170 to 180, at least from about 180 to 190, at least from about 190 to 200. In a more preferred embodiment, said ratio is 1 µg of nucleic acid/0.3 nM of nanoparticles. In another preferred embodiment, said ratio is 3 µg of nucleic acid/0.3 nM of nanoparticles. In another preferred embodiment, said ratio is 3 µg of nucleic acid/0.3 nM of nanoparticles.

The person skilled in the art will understand that said molar ratio can vary depending of the nature of the nucleic acid, i.e. double stranded or single stranded nucleic acid. By way of illustration, the aggregate of the invention is formed by putting in contact metallic nanoparticles in an appropriate concentration (i.e. 0.3 nM) with an appropriate concentration of single stranded nucleic acid molecules (i.e. within 1x10⁻⁷ M to 1x10⁻⁶ M) during an appropriate contact time, for example 3 hours. In another preferred embodiment, the aggregate of the invention is formed by putting in contact metallic nanoparticles in an appropriate concentration (i.e. 0.3 nM) with an appropriate concentration of double stranded nucleic acid molecules (i.e. within 5x10⁻⁷ M to 5x10⁻⁸ M) during an appropriate contact time, for example 3 hours.

In a preferred embodiment of the invention, said nucleic acid is double stranded DNA. In another preferred embodiment of the invention, said nucleic acid is single stranded DNA. In another preferred embodiment of the invention, said nucleic acid comprises both, double stranded DNA and single stranded DNA molecules. Depending on the enrichment in double stranded or in single stranded DNA molecules, the molar ratio can vary among values 0 and 1. In this event, the person skilled in the art will understand that the molar ratio of metal nanoparticles to double and single stranded DNA molecules should also be adjusted in order to avoid large formation of unstable aggregates (high nanoparticle-to-nucleic acid ratios) or limited formation of stable aggregates (low nanoparticle-to-nucleic acid ratios). By way of illustration, suitable molar ratios of single double stranded DNA molecules with respect to double stranded DNA molecules in the aggregates include 0.011, 0.024, 0.041, 0.063, 0.091, 0.130, 0.189, 0.286, 0.474 and 1M.

### Method for detecting the presence of a nucleic acid in a sample

In another aspect the invention relates to a method for detecting the presence of a nucleic acid in a sample, hereinafter referred as "the second method of the invention", comprising the steps of:
(i) contacting said sample with a population of metallic nanoparticles, wherein said metallic nanoparticles are coated with a polycation wherein if a nucleic acid is present in said sample then said metallic nanoparticles form aggregates that are stabilized by electrostatic interactions between the negative charges in said nucleic acid and the positive charges of the polycation; and
(ii) obtaining a SERS spectrum of the sample
wherein an increase in the band intensity characteristic of a purine or pyrimidine base in a nucleic acid forming part of the aggregate with respect to said band intensity characteristic of a purine or pyrimidine base in a nucleic acid which does not form part of the aggregate is indicative of the presence of a nucleic acid in the sample.

The terms "population of metallic nanoparticles", "metallic nanoparticles" "polycation", "electrostatic interaction", "aggregate" and "nucleic acid" as their particular and preferred embodiments thereof have been defined in the context of the first aspect of the invention and equally apply to the second method of the invention.

In the first step, the second method of the invention comprises contacting the sample of interest with a population of metallic nanoparticles coated with a polycation. Said first step must take place under conditions that allow the electrostatic interaction between the negative charges of the phosphate group of the nucleic acid, if present in the sample, and the positive charges of the polycation that coat the metallic nanoparticles. Said conditions under which the first step of the second method of the invention is carried out will be those conditions in which spontaneous nanoparticle aggregation does not occur, i.e., those conditions in which in absence of the nucleic acid target molecule to be detected, the nanoparticles are in suspension and individualized. As has been mentioned above, the person skilled in the art will understand that suitable conditions that allow said electrostatic interaction are those which do not modify the negative charge of the nucleic acid and which do not modify the positive charge of the polycation in the coats of the metallic nanoparticles. Said conditions include an appropriate pH, appropriate ion strength, an appropriate temperature, an appropriate concentration of a nucleic acid and appropriate conditions of salt, pH, temperature and time of contacting. If the sample comprises nucleic acids, the consequence of the electrostatic interaction between the negative charges of the nucleic acid molecules present in the sample and the positive charges of the polycation in the coats of said metal nanoparticles is the formation of aggregates formed by long-term-stable clusters in suspension where the nucleic acid molecules are trapped within inter-nanoparticle junctions. Suitable conditions for carry out the first step of the second method of the invention are illustrated in the examples of the present application.

The second step of the second method of the invention comprises obtaining a SERS spectrum of the sample. The term "SERS" or "Surface Enhanced Raman Scattering" as used herein, refers to a surface sensitive technique that results in the enhancement of Raman scattering by molecules adsorbed on metal surfaces. The term "SERS spectrum" as used herein refers to a SERS spectrum comprised in the spectral region between 100 to 3500 cm⁻¹. Typically, a spectrum represents intensities depending on energy. The energy scale is expressed in frequencies, intensity ratio, wavelength and a peak area. Normally, the analysis of the obtained SERS spectrum is carried out by deconvoluting the spectrum, i.e. dividing the spectrum into individual peaks contribution to the whole spectrum. The SERS spectrum can be obtained by using an appropriate spectrophotometer. The SERS spectrum is typically reported in wavenumbers, which have units of inverse length as this value is directly related to energy. In order to convert between the spectral wavelength and wavenumbers on shift in the SERS spectrum, the following formula can be used: Δω= (1/λ₀ - 1/λ₁); wherein Δω is the shift expressed in wavenumber, λ₀ is the excitation wavelength, and λ₁ is the SESR spectrum wavelength. Most commonly, the unit for expression wavenumber is cm⁻¹. Normally, the abcisa of SERS spectrum is the wavenumber "shift" which is defined as the difference in wavenumbers between the incident radiation and the scattered radiation. Normally, the ordinate axis of the SESR spectrum represents the intensity of the bands. The frequencies may be used to identify the composition of a sample. If, for example, intensities are plotted on a Y-axis, and frequency or frequencies are plotted on an X-axis, the frequency or frequencies may be expressed as a wave number, the reciprocal of the wavelength expressed in centimeters. The X-axis, showing frequency or frequencies, may be converted to a Raman shift wave numbers, the measure of the difference between the observed wave number position of spectral bands, and the wave number of radiation appearing in the incident radiation.

In a preferred embodiment, the metal nanoparticles which are used in the second method of the invention are silver nanoparticles or gold nanoparticles. Thus, in the event that the nanoparticles are silver nanoparticles the determination of the SERS spectra can be carried out by using a 514 or 532 nm laser. The spectrum is obtained after the laser with the appropriate wavelength is focused onto the sample during a time interval that is considered suitable. For example, the spectrum can be acquired after a exposure time of 1 second, 2 seconds, 3 seconds, 4 seconds, 5 seconds, 6 seconds, 7 seconds, 8 seconds, 9 seconds, 10 seconds or more.

According to the second method of the invention, the detection of a nucleic acid in the given sample can be determined by comparing the SERS spectrum obtained in the second step of said method with a normal Raman spectrum obtained from a sample comprising nucleic acids which does not form part of said aggregate, that is to say, in absence of the metallic nanoparticles defined in the first step of the present method. Specifically, an increase in the intensity of a band characteristic of a purine or pyrimidine base in a nucleic acid forming part of the aggregate with respect to said band intensity characteristic of a purine or pyrimidine base in a nucleic acid which does not form part of the aggregate is indicative of the presence of a nucleic acid in the sample. The term "band intensity characteristic of a purine or pyrimidine base in a nucleic acid forming part of the aggregate", as used herein refers to those spectral bands which are comprised between about 503 cm⁻¹ and about 2960 cm⁻¹.

The obtention of the normal Raman spectrum from a sample comprising nucleic acids but in absence of metallic nanoparticles as defined in the first step of the invention will preferably carried out in the same conditions that those conditions under which the SERS spectrum of the sample to be analyzed is obtained. In a preferred embodiment of the invention, the obtention of the SERS spectrum in a sample containing the nucleic acid to be detected is carried out at the same time as the obtention of said normal Raman spectrum of the nucleic acid in a sample not containing the nanoparticles as previously defined. Alternatively, the invention contemplates the obtention of the spectrum of both samples being determined at different times spaced out by a suitable time interval; for example, 1 hour, 2 hours, 5 hours, 10 hours, 12 hours, 24 hours, two days, three days, five days, ten days, fifteen days, a month, six months, a year or more can lapse between the determination of SERS spectrum the sample to be detected and the determination of the normal Raman spectrum in a sample containing a nucleic acid which does not form part of an aggregate. If the obtention of the spectrum in said samples is carried out at times spaced out by a time interval, then it is advisable to store the nanoparticles under suitable conditions to avoid their degradation.

As it is used herein, the term "an increase in the band intensity" refers to an at least 0.01-fold, 0.05-fold, 0.075-fold, 0.1-fold, 0.5-fold, 1-fold, 1.5-fold, 2-fold, 5-fold, 10-fold, 15-fold, 20-fold, 30-fold, 40-fold, 50-fold, 60-fold, 70-fold, 80-fold, 90-fold, or 100-fold increase in the band intensity with respect to said band intensity measured in nucleic acid which does not form part of the aggregate. Thus in the context of the present method, said term must be understood as at least 0.01-fold, 0.05-fold, 0.075-fold, 0.1-fold, 0.5-fold, 1-fold, 1.5-fold, 2-fold, 5-fold, 10-fold, 15-fold, 20-fold, 30-fold, 40-fold, 50-fold, 60-fold, 70-fold, 80-fold, 90-fold, or 100-fold increase in the band intensity characteristic of a purine or pyrimidine base in a nucleic acid forming part of the aggregate with respect to said band intensity of the sample which comprises a nucleic acid which does not form part of the aggregate.

As it is used herein, the term "sample" refers to any material containing nucleic acid, for example double or single stranded DNA or double or single stranded RNA which is obtained from a biological sample or artificially synthesized. In a particular embodiment, said nucleic acid forms part of a biological sample. As it is used herein, "biological sample" refers to a tissue-, cellular- or biological fluid-type material. If the material in which the presence of a certain nucleic acid is to be determined according to the present method is a tissue or a cell, prior extraction of the nucleic acid from the sample is preferably performed using any technique suitable for that purpose. The biological sample can be treated to physically or mechanically break down the tissue or cell structure, releasing the intracellular components into an aqueous or organic solution to prepare the DNA or RNA. In a particular embodiment of the invention, said biological sample is a cell lysate. In another particular embodiment, said biological sample is a cell lysate. In another particular embodiment, said biological sample is a biological fluid. RNA extraction can be performed by any of the methods known by the person skilled in the art, including, without being limited to, Trizol®, guanidinium salts, phenol, chloroform, etc. There are also commercial kits that allow extracting RNA from a sample, such as the Qiagen® RNA extraction kit, for example. As the person skilled in the art knows, maximum precautions must be taken when working with RNA to avoid contaminations with RNases and RNA degradation. After obtaining the RNA, an mRNA reverse transcription (RT) reaction followed by amplification by polymerase chain reaction (PCR) [RT-PCR] can be carried out if desired in order to obtain the double helix cDNA corresponding to the RNA present in the sample. As it is used herein, the term "cDNA" or "complementary DNA" refers to the single-stranded DNA that is synthesized from a single strand of RNA. As the person skilled in the art can understand, there are several cDNA synthesis methods, the most common being the use of the enzyme reverse transcriptase. Methods for carrying out cDNA synthesis are well known in the state of the art.

In a particular embodiment of the invention, the nucleic acid to be determined is double stranded DNA. In another particular embodiment of the invention, the nucleic acid to be determined is single stranded DNA. In another particular embodiment of the invention, the nucleic acid to be determined is double stranded RNA or single stranded RNA.

In a particular and preferred embodiment, the second method of the invention further comprises a step of normalization of the SERS spectrum using the peak height of the band at 1090 cm⁻¹. The peak height at 1090 cm⁻¹ is approximately proportional to the number of phosphate groups in the sequence, either single or double-stranded.

The second method of the invention allows the determination of the type of nucleic acid (if any) present in the sample under study depending on the intensity of the band characteristic of a purine or pyrimidine.

Therefore, in a particular embodiment of the invention, if the increase in the intensity of the band characteristic of a purine or pyrimidine base in a nucleic acid forming part of the aggregate with respect to said band intensity characteristic of a purine or pyrimidine base in a nucleic acid which does not form part of the aggregate wherein the band is selected from the group consisting of a band at about 503 cm⁻¹, at about 621 cm⁻¹, at about 665/677 cm⁻¹, at about 730 cm⁻¹, at about 752 cm⁻¹, at about 787 cm⁻¹, at about 1019 cm⁻¹, at about 1324 cm⁻¹, at about 1653 cm⁻¹, at about 2806 cm⁻¹ and at about 2967 cm⁻¹, then the nucleic acid is double stranded DNA.

In another particular embodiment of the invention, if the increase in the intensity of the band characteristic of a purine or pyrimidine base in a nucleic acid forming part of the aggregate with respect to said band intensity characteristic of a purine or pyrimidine base in a nucleic acid which does not form part of the aggregate wherein the band is selected from the group consisting of a band at about 512 cm⁻¹, about 686 cm⁻¹, at about 734 cm⁻¹, at about 793 cm⁻¹, at about 1029 cm⁻¹, at about 1199 cm⁻¹, at about 1329 cm⁻¹, at about 1643 cm⁻¹ and at about 2960 cm⁻¹, then the nucleic acid is single stranded DNA.

In another particular embodiment of the invention, if the increase in the intensity of the band characteristic of a purine or pyrimidine base in a nucleic acid forming part of the aggregate with respect to said band intensity characteristic of a purine or pyrimidine base in a nucleic acid which does not form part of the aggregate wherein the band is selected from the group consisting of a band at about 599 cm⁻¹, at about 1090 cm⁻¹, at about 1178 cm⁻¹, at about 1246/1264 cm⁻¹, at about 1354 cm⁻¹, at about 1376 cm⁻¹, at about 1421 cm⁻¹, at about 1487 cm⁻¹, at about 1509 cm⁻¹, at about 1528 cm⁻¹, at about 1577 cm⁻¹ and at about 1628 cm⁻¹, then the nucleic acid is single stranded RNA or double stranded RNA.

### Method for detecting the presence of a given nucleotide at predetermined position in a target nucleic acid

In another aspect, the invention relates to a method for detecting the presence of a given nucleotide at a predetermined position in a target nucleic acid, hereinafter "the third method of the invention", comprising the steps of:
(i) contacting a population of metallic nanoparticles coated with a polycation separately with the target nucleic acid and with a control nucleic acid having the same sequence as the target nucleic acid and having a known nucleotide at said predetermined position, thereby resulting in the formation of a first type of aggregates comprising the metallic nanoparticles and the target nucleic acid and a second type of aggregates comprising the metallic nanoparticles and the control nucleic acid,
(ii) obtaining the SERS spectra of the first and second types of aggregates obtained in step (i); and
wherein if the SERS spectrum of the first type of aggregates and the SERS spectrum of the second type of aggregates are substantially identical, then the nucleotide at said predetermined position in the target nucleic acid is the same as the known nucleotide or
wherein if the SERS spectrum of the first type of aggregates and the SERS spectrum of the second type of aggregates are the SERS spectrum are different, then the nucleotide at said predetermined position is different from the known nucleotide.

The terms "population of metallic nanoparticles", "polycation", "electrostatic interaction", "aggregate" and "nucleic acid" as their particular and preferred embodiments thereof have been defined in the context of the first and second aspects of the invention and equally apply to this aspect of the invention.

The first step of the third method of the invention comprises contacting a population of metallic nanoparticles coated with a polycation with the target nucleic acid. The term "target nucleic acid", as used herein, refers to a sequence of a nucleic acid formed by at least one nucleotide. In a preferred embodiment of the invention, the target sequence to be detected is a nucleotide. In another preferred embodiment, the target sequence to be detected is formed by 2, 3, 4, 5, 10, 15, 20, 30, 40, 50 or more nucleotides. As has been explained above in the context of the second method of the invention, the result of putting in contact nucleic acid molecules with metallic nanoparticles coated with a polycation is the formation of aggregates due to the electrostatic interaction between the negative charges of the phosphate groups of the nucleic acid and the positive charges of the polycation which coat the metallic nanoparticles. Thus, according to the first step of the third method of the invention, the contact of a population of metallic nanoparticles coated with a polycation with said target nucleic acid results in the formation of a first type of aggregates.

The first step of the third method of the invention also comprises contacting a population of metallic nanoparticles coated with a polycation with a second sample, namely, with a control nuclei acid. The term "control nucleic acid" as used herein refers to a sequence of a nucleic acid having the same sequence as the target nucleic acid and having a known nucleotide at the predetermined position to be detected in the target nucleic acid. The result of the electrostatic interaction between the negative charge of the control nucleic acid and the positive charge of the polycation in the coats of said nanoparticles is the formation of a second type of aggregates.

Contacting the target nucleic acid with a population of metallic nanoparticles coated with a polycation according to the invention is done separately with respect to contacting said metallic nanoparticles with the control nucleic acid sample. In preferred embodiments of the invention, the first step comprises contacting an aliquot of metallic nanoparticles coated with a polycation with the target nucleic acid to be detected giving rise to said first type of aggregates, and separately contacting another aliquot of the metallic nanoparticles coated with a polycation with the control nucleic acid giving rise to said second type of aggregates, wherein said aliquot is different from the aliquot that is contacted with the target nucleic acid. Similar concentration of nanoparticles in said different aliquots is preferred. Methods for determining the concentration of nanoparticles are mentioned in the context of the first method of the invention.

Said first step is carried out under conditions that allow electrostatic interaction between said target nucleic acid and said nanoparticles coated with a polycation and between said control nucleic acid and said nanoparticles coated with a polycation forming the first and the first type of aggregates. Suitable conditions that allow said electrostatic interaction has been previously mentioned in the context of the second method of the invention. It is preferred using the same suitable conditions that allow the formation of said first and second type of aggregates. As has been previously mentioned in the context of the first method of the invention, the ratio of nanoparticles to the nucleic acid must be carefully adjusted in order to avoid large formation of unstable aggregates (high nanoparticle-to-nucleic acid ratios) or limited formation of stable aggregates (low nanoparticle-to-nucleic acid ratios). Thus, the ratio of the concentration of the metallic nanoparticles coated with a polycation to the concentration of the target nucleic acid and the ratio of the concentration of the metallic nanoparticles coated with a polycation to the concentration of the control nucleic acid must be adjusted.

In a preferred embodiment, said ratio between metallic nanoparticles/nucleic acid refers to a molar ratio between said metallic nanoparticles/nucleic acid. In a preferred embodiment, said molar ratio is at least from about 1:1000 to about 1000:1, at least from about 1:900 to 900:1, at least from about 1:800 to 800:1, at least from about 1:700 to 700:1, at least from about 1:600 to 600:1, at least from about 1:500 to 500:1, at least from about 1:400 to 400:1, at least from about 1:300 to 300:1, at least from about 1:200 to 200:1, at least from about 1:100 to 100:1, at least from about 1:90 to 90:1, at least from about 1:80 to 80:1, at least from about 1:70 to 70:1, at least from about 1:60 to 60:1, at least from about 1:50 to 50:1, at least from about 1:40 to 40:1, at least from about 1:30 to 30:1, at least from about 1:20 to 20:1, at least from about 1:10 to 10:1, at least from about 1:5 to 5:1, at least from about 1:2 to 2:1. In another preferred embodiment said molar ratio is 1:100. In another preferred embodiment said molar ratio is 1:103 or more. In a more preferred embodiment said molar ratio is 1:1000. In another preferred embodiment said molar ratio is 1:166. In another preferred embodiment said molar ratio is 1:3. In another preferred embodiment said molar ratio is 1:833.

In another preferred embodiment, said ratio refers to the weight of said nucleic acid to molar concentration of said metallic nanoparticles. In this event, in a preferred embodiment the ratio is at least from about 0.1 to 1, at least from about 1 to 5, at least from about 5 to 10, at least from about 10 to 20, at least from about 20 to 30, at least from about 30 to 40, at least from about 40 to 50, at least from about 50 to 60, at least from about 60 to 70, at least from about 70 to 80, at least from about 80 to 90, at least from about 90 to 100, at least from about 100 to 110, at least from about 110 to 120, , at least from about 120 to 130, at least from about 130 to 140, , at least from about 140 to 150, at least from about 150 to 160, at least from about 160 to 170, at least from about 170 to 180, at least from about 180 to 190, at least from about 190 to 200. In a more preferred embodiment, said ratio is 1 µg of nucleic acid/0.3 nM of nanoparticles. In another preferred embodiment, said ratio is 3 µg of nucleic acid/0.3 nM of nanoparticles. In another preferred embodiment, said ratio is 3 µg of nucleic acid/0.3 nM of nanoparticles.Suitable concentrations of nanoparticles and nucleic acids which can be used according to the present method are detailed in the examples of the present application.

The second step of the third method of the invention comprises obtaining the SERS spectra of the first type and the second type of aggregates obtained in the first step of said method. The term "SERS spectrum" has been previously defined. According to the present method, the presence of a given nucleotide at a predetermined position in a target sequence is determined by the SERS spectrum differences between:
(i) a first type of aggregates according to the invention, wherein said first type of aggregates are formed by metallic nanoparticles coated with a polycation and the target nucleic acid;
(ii) a second type of aggregates according to the invention, wherein said second type of aggregates are formed by metallic nanoparticles coated with a polycation and the control nucleic acid

According to the present method, if the SERS spectrum of the first type of aggregates and the SERS spectrum of the second types of aggregates are substantially identical, then the nucleotide at said determined position in the target nucleic acid is the same as the known nucleotide. The term "SERS substantially identical" in the context of the present invention means that the intensity of at least one band in the SERS spectrum of the first type of aggregate differs less than 50%, less than 40%, less than 30%, less than 20%, less than 10%, less than 5%, less than 4%, less than 3%, less than 2%, less than 1%, less than 0.5% or less from the intensity of the said at least band in the SERS spectrum of the second type of aggregates. By said term is also understood that the shift of at least one band in the SERS spectrum of the first type of aggregate differs less than 50%, less than 40%, less than 30%, less than 20%, less than 10%, less than 5%, less than 4%, less than 3%, less than 2%, less than 1%, less than 0.5% or less from the shift of said at least one band in the SERS spectrum of the second type of aggregates.

On the contrary, if the SERS spectrum of the first type of aggregates and the SERS spectrum of the second types of aggregates are different, then the nucleotide at said determined position in the target nucleic acid is different from the known nucleotide. The term "SERS different" in the context of the present invention means that the intensity of at least one band in the SERS spectrum of the first type of aggregate differs more than 51%, more than 60%, more than 70%, more than 80%, more than 90%, more than 91%, more than 92%, more than 93%, more than 94%, more than 95%, more than 99% or more from the intensity of the said at least band in the SERS spectrum of the second type of aggregates. By said term is also understood that that the shift of at least one band in the SERS spectrum of the first type of aggregate differs more than 51%, more than 60%, more than 70%, more than 80%, more than 90%, more than 91%, more than 92%, more than 93%, more than 94%, more than 95%, more than 99% or more from the shift of said at least one band in the SERS spectrum of the second type of aggregates.

According to the third method of the invention, it is possible to identify whether the given predetermined position is adenine, guanine, cytosine thymine or uracil based on the vibrational SERS spectrum associated with each of said nucleic bases.

Thus, in a more particular embodiment of the third method of the invention, if the difference between the spectra of the first and second types of aggregates is an increase in the intensity of a band selected from the group consisting of a band at 730 cm⁻¹, at 734 cm⁻¹, at 1224 cm⁻¹, at 1329 cm⁻¹, at 1508 cm⁻¹ and 1577 cm⁻¹, then it is indicative that the nucleotide at said predetermined position is adenine.

In another particular embodiment of the third method of the invention, if the difference between the spectra of the first and second types of aggregates is an increase in the intensity of a band at 1577 cm⁻¹, then it is indicative that the nucleotide at said predetermined position is adenine or guanine.

In another particular embodiment of the third method of the invention, if the difference between the spectra of the first and second types of aggregates is an increase in the intensity of a band selected from the group consisting of a band at 621 cm⁻¹, at 665/677 cm⁻¹, at 686 cm⁻¹, at 1354 cm⁻¹, at 1487 cm⁻¹, then it is indicative that the nucleotide at said predetermined position is guanine.

In another particular embodiment of the third method of the invention, if the difference between the spectra of the first and second types of aggregates is an increase in the intensity of a band selected from the group consisting of a band at 787 cm⁻¹ and at 793 cm⁻¹, then it is indicative that the nucleotide at said predetermined position is cytosine or thymine.

In another particular embodiment of the third method of the invention, if the difference between the spectra of the first and second types of aggregates is an increase in the intensity of a band selected from the group consisting of a band at 1178 cm⁻¹, at 1376 cm⁻¹, at 1643 cm⁻¹ and at 1653 cm⁻¹, then it is indicative that the nucleotide at said predetermined position is thymine.

In another particular embodiment of the third method of the invention, if the difference between the spectra of the first and second types of aggregates is an increase in the intensity of a band selected from the group consisting of a band at 1246/1264 cm⁻¹ and 1528 cm⁻¹, then it is indicative that the nucleotide at said predetermined position is cytosine.

In another particular embodiment of the third method of the invention, if the difference between the spectra of the first and second types of aggregates is an increase in the intensity of a band selected from the group consisting of a band at 1274 cm⁻¹ and 1630 cm⁻¹, then it is indicative that the nucleotide at said predetermined position is uracil.

In a particular embodiment of the third method of the invention, the target nucleic acid is a single stranded nucleic acid and the control nucleic acid is a double stranded nucleic acid. In an even more particular embodiment of the invention, wherein the target nucleic acid is a single stranded nucleic acid, the first step of the present method is preceded by a step of contacting the target nucleic acid with a probe nucleic acid having a sequence which is fully complementary to the sequence of the target nucleic acid in the region comprising said predetermined position with the exception of the nucleotide at the predetermined position which contains a nucleotide different to the nucleotide complementary to said given nucleotide and wherein the control nucleic acid is a double stranded nucleic acid having a first strand the sequence of which has the same sequence as the target nucleic acid and having a known nucleotide at said predetermined position and a second strand which is fully complementary with the target nucleic acid.

The term "complementary", when used to describe a first nucleotide sequence in relation to a second nucleotide sequence, refers to the ability of an oligonucleotide or polynucleotide comprising the first nucleotide sequence (i.e. the sequence of the target nucleic acid) to hybridize and form a duplex structure under certain conditions with an oligonucleotide or polynucleotide comprising the second nucleotide sequence (i.e. the probe nucleic acid), as will be understood by the skilled person. The person skilled in the art can empirically determine the stability of a duplex taking a number of variables into account, such as probe base pair length and concentration, ionic strength and mismatched base pair incidence, following the guidelines of the state of the art.

The term "probe" or "probe nucleic acid", as used herein, refers to an oligonucleotide that is capable of forming a duplex structure by complementary base pairing with a sequence of a target polynucleotide and is generally not able to form primer extension products. The probe nucleic acid according to this embodiment of the invention is characterized in that it has a sequence which is fully complementary to the sequence of the target nucleic acid in the region comprising said predetermined position with the exception of the nucleotide at the predetermined position which contains a nucleotide different to the nucleotide complementary to said given nucleotide.

Said previous step preceding the first step of the third method of the invention must take place under conditions that allow hybridizing said nucleic acid probe with the target nucleic acid. Such conditions can, for example, be stringent conditions, where stringent conditions may include: 400 mM NaCl, 40 mM PIPES pH 6.4, 1 mM EDTA, 50 degrees centigrade or 70 degrees centigrade for 12-16 hours followed by washing. Other conditions, such as physiologically relevant conditions as may be encountered inside an organism, can apply. The skilled person will be able to determine the set of conditions most appropriate for a test of complementarity of two sequences in accordance with the ultimate application of the hybridized nucleotides. Preferably, after the hybridization occurs, the double stranded DNA is purified before carry out the first step of the third method of the invention.

In a still more particular embodiment, the position comprising the given nucleotide is located terminally in the double stranded nucleic acid. In another particular embodiment the position comprising the given nucleotide is located within the strand on the nucleic acid.

In a still more particular and preferred embodiment, the third method of the invention further comprises a step of normalization of the SERS spectra using the peak height of the band at 1090 cm⁻¹.

In a more particular embodiment of the third method of the invention, the target nucleic acid is a substantially isolated nucleic acid molecule. The term "substantially isolated nucleic acid molecule" as used herein, refers to a nucleic acid molecule that preferably contains no sequences which naturally flank the nucleic acid in the genomic DNA of the organism from which the nucleic acid originates. The nucleic acid molecules may be isolated using standard techniques of molecular biology and the sequence information provided herein. Using comparative algorithms, it is possible to identify for example a homologous sequence, or homologous, conserved sequence regions, at the DNA or amino acid level. Essential portions of this sequence or the entire homologous sequence can be used as hybridization probe using standard hybridization techniques for isolating further nucleic acid sequences which are useful in the method from other organisms by screening cDNA libraries and/or genomic libraries. Said term also means that the nucleic acid molecule is essentially free of cellular contaminants such cell remains, proteins, lipids, carbohydrates, glycoproteins, glycolipids etc.

Moreover, a nucleic acid molecule or a part thereof can be isolated by means of polymerase chain reaction ("PCR"), where oligonucleotide primers based on the sequences specified herein or parts thereof are used (for example, it is possible to isolate a nucleic acid molecule comprising the complete sequence or part thereof by means of PCR using oligonucleotide primers which have been generated on the basis of the very same sequence). For example, mRNA can be isolated from cells (for example by the guanidinium thiocyanate extraction method) and cDNA prepared therefrom by means of reverse. A nucleic acid can be amplified using cDNA or, alternatively, genomic DNA as template and suitable oligonucleotide primers by means of standard PCR amplification techniques. The nucleic acid amplified thus can be cloned into a suitable vector and characterized by means of DNA sequence analysis. Oligonucleotides which correspond to a nucleotide sequence coding for a protein can be prepared by synthetic standard methods, for example, using an automated DNA synthesizer.

If desired, the target nucleic acid and/or the control nucleic acid can be subjected to a previous step of amplification (enrichment) before carry out the first step of the third method of the invention. This is recommendable, although not necessary, if the amount of the target nucleic acid and/or the control nucleic acid is small (i.e. about picograms) in order to obtain a higher number of copies of said nucleic acid. Methods for amplifying nucleic acid molecules are known in the art, and include, but are not limited to, polymerase chain reaction (PCR), multiple displacement amplification (MDA), ligase chain reaction (LCR), Q-replicase amplification, polymerase chain reaction (PCR), degenerate oligonucleotide primed-polymerase chain reaction (DOP-PCR), rolling circle amplification (RCA), T7/primase-dependent amplification, strand-displacement amplification (SDA), self-sustained sequence replication (3SR), nucleic acid sequence-based amplification (NASBA) and loop-mediated isothermal amplification (LAMP). If this step of amplification is carried out, purified products are preferred to carry out the first step of the third method of the invention.

### Method for detecting the presence of a modified nucleotide at a predetermined position in a target nucleic acid

In another aspect, the invention relates to a method for detecting the presence of a modified nucleotide at a predetermined position in a target nucleic acid, hereinafter, "the fourth method of the invention" comprising the steps of:
(i) contacting a population of metallic nanoparticles coated with a polycation separately with the target nucleic acid and with a control nucleic acid having the same sequence as the target nucleic acid and wherein the predetermined position is not modified, thereby resulting in the formation of a first type of aggregates comprising the metallic nanoparticles and the target nucleic acid and a second type of aggregates comprising the metallic nanoparticles and the control nucleic acid; and;
(ii) obtaining the SERS spectra of the first and second types of aggregates obtained in step (i)
wherein if the SERS spectrum of the first type of aggregates and the SERS spectrum of the second type of aggregates are substantially identical, then the nucleotide at said predetermined position is not modified or
wherein if the SERS spectrum of the first type of aggregates and the SERS spectrum of the second type of aggregates are different, then the nucleotide at said predetermined position is modified.

The terms "population of metallic nanoparticles", "polycation", "electrostatic interaction", "aggregate", and "nucleic acid" as the particular and preferred embodiments thereof have been defined in the context of the first and second aspects of the invention and equally apply to this aspect of the invention.

The term "modified nucleotide", as used herein, relates to nucleotides with a covalently modified base and/or sugar. For example, modified nucleotides include nucleotides having sugars which are covalently attached to low molecular weight organic groups other than a hydroxyl group at the 3' position and other than a phosphate group at the 5' position. Thus modified nucleotides may also include 2' substituted sugars such as 2'-O-methyl-; 2-O-alkyl; 2-O-allyl; 2'-S-alkyl; 2'-S-allyl; 2'-fluoro-; 2'-halo or 2-azido-ribose, carbocyclic sugar analogues a-anomeric sugars; epimeric sugars such as arabinose, xyloses or lyxoses, pyranose sugars, furanose sugars, and sedoheptulose. Modified nucleotides are known in the art and include, by example and not by way of limitation, alkylated purines and/or pyrimidines; acylated purines and/or pyrimidines; or other heterocycles. These classes of pyrimidines and purines are known in the art and include, pseudoisocytosine; N4, N4-ethanocytosine; 8-hydroxy-N6-methyladenine; 4-acetylcytosine, 5-(carboxyhydroxylmethyl) uracil; 5-fluorouracil; 5-bromouracil; 5-carboxymethylaminomethyl-2-thiouracil; 5-carboxymethylaminomethyl uracil; dihydrouracil; inosine; N6-isopentyl-adenine; 1-methyladenine; 1-methylpseudouracil; 1-methylguanine; 2,2-dimethylguanine; 2-methyladenine; 2-methylguanine; 3-methylcytosine; 5-methylcytosine; N6-methyladenine; 7-methylguanine; 5-methylaminomethyl uracil; 5-methoxy amino methyl-2-thiouracil; β-D-mannosylqueosine; 5-methoxycarbonylmethyluracil; 5-methoxyuracil; 2-methylthio-N6-isopentenyladenine; uracil-5-oxyacetic acid methyl ester; psueouracil; 2-thiocytosine; 5-methyl-2 thiouracil, 2- thiouracil; 4-thiouracil; 5-methyluracil; N-uracil-5-oxyacetic acid methylester; uracil 5-oxyacetic acid; queosine; 2-thiocytosine; 5-propyluracil; 5-propylcytosine; 5-ethyluracil; 5-ethylcytosine; 5-butyluracil; 5-pentyluracil; 5-pentylcytosine; and 2,6,-diaminopurine; methylpsuedouracil; 1-methylguanine; 1-methylcytosine.

In a particular embodiment, said modification is selected from the group consisting of a 5-methyl Cytosine, a 5-hydroxymethyl Cytosine, a 5-X Cytosine, wherein X is Cl or Br, a N6-methyl Adenine, a 8-oxo Guanine, a cyclobutane pyrimidine dimer and a 6-4 photoproduct.

The first step of the fourth method of the invention comprises contacting a population of metallic nanoparticles coated with a polycation with the target nucleic acid. The term "target nucleic acid" has been defined in the context of the third method of the invention as is used herein with the same meaning. As has been previously explained, the result of putting in contact a nucleic acid, i.e. the target nucleic acid, which is negatively charged with a population of metallic nanoparticles coated with a polycation which is positively charged, is the formation of aggregates, namely the first type of aggregates according to the fourth method of the invention.

The first step of the fourth method of the invention also comprises contacting said population of metallic nanoparticles coated with a polycation with a second sample, namely with a control nuclei acid. The term "control nucleic acid" as used herein refers to a sequence of a nucleic acid having the same sequence as the target nucleic acid and wherein the predetermined position to be detected in the target nucleic acid is not modified. The result of the electrostatic interaction between the negative charge of the control nucleic acid and the positive charge of the polycation in the coats of said nanoparticles is the formation of a second type of aggregate.

Contacting the target nucleic acid with metallic nanoparticles coated with a polycation according to the invention is done separately with respect to contacting said metallic nanoparticles with the control nucleic acid sample. In preferred embodiments of the invention, the first step comprises contacting an aliquot of metallic nanoparticles coated with a polycation with the target nucleic acid to be detected giving rise to said first type of aggregates, and separately contacting another aliquot of the metallic nanoparticles coated with a polycation with the control nucleic acid giving rise to said second type of aggregates, wherein said aliquot is different from the aliquot that is contacted with the target nucleic acid. Similar concentration of nanoparticles in said different aliquots is preferred. Methods for determining the concentration of nanoparticles are mentioned in the context of the second method of the invention.

The first step of the fourth method of the invention must be carried out under conditions that allow electrostatic interaction between said target nucleic acid and said metallic nanoparticles coated with a polycation and between said control nucleic acid and said nanoparticles forming the first and the first type of aggregates. Suitable conditions that allow said electrostatic interaction has been previously mentioned in the context of the second method of the invention. Suitable conditions that allow the formation of said first type of aggregates are used as preferred conditions that allow the formation of said second type of aggregates. As has been previously mentioned, the molar ratio of nanoparticles to the nucleic acid must be carefully adjusted in order to avoid large formation of unstable aggregates (high nanoparticle-to-nucleic acid ratios) or limited formation of stable aggregates (low nanoparticle-to-nucleic acid ratios). Thus, the ratio of the concentration of the metallic nanoparticles coated with a polycation to the concentration of the target nucleic acid and the ratio of the concentration of the metallic nanoparticles coated with a polycation to the concentration of the control nucleic acid must be adjusted in order to form the first and the second types of aggregates according to the fourth method of the invention.

In a preferred embodiment, said ratio between metallic nanoparticles/nucleic acid refers to a molar ratio between said metallic nanoparticles/nucleic acid. In a preferred embodiment, said molar ratio is at least from about 1:1000 to about 1000:1, at least from about 1:900 to 900:1, at least from about 1:800 to 800:1, at least from about 1:700 to 700:1, at least from about 1:600 to 600:1, at least from about 1:500 to 500:1, at least from about 1:400 to 400:1, at least from about 1:300 to 300:1, at least from about 1:200 to 200:1, at least from about 1:100 to 100:1, at least from about 1:90 to 90:1, at least from about 1:80 to 80:1, at least from about 1:70 to 70:1, at least from about 1:60 to 60:1, at least from about 1:50 to 50:1, at least from about 1:40 to 40:1, at least from about 1:30 to 30:1, at least from about 1:20 to 20:1, at least from about 1:10 to 10:1, at least from about 1:5 to 5:1, at least from about 1:2 to 2:1. In another preferred embodiment said molar ratio is 1:100. In another preferred embodiment said molar ratio is 1:103 or more. In a more preferred embodiment said molar ratio is 1:1000. In another preferred embodiment said molar ratio is 1:166. In another preferred embodiment said molar ratio is 1:3. In another preferred embodiment said molar ratio is 1:833.

In another preferred embodiment, said ratio refers to the weight of said nucleic acid to molar concentration of said metallic nanoparticles. In this event, in a preferred embodiment the ratio is at least from about 0.1 to 1, at least from about 1 to 5, at least from about 5 to 10, at least from about 10 to 20, at least from about 20 to 30, at least from about 30 to 40, at least from about 40 to 50, at least from about 50 to 60, at least from about 60 to 70, at least from about 70 to 80, at least from about 80 to 90, at least from about 90 to 100, at least from about 100 to 110, at least from about 110 to 120, , at least from about 120 to 130, at least from about 130 to 140, , at least from about 140 to 150, at least from about 150 to 160, at least from about 160 to 170, at least from about 170 to 180, at least from about 180 to 190, at least from about 190 to 200. In a more preferred embodiment, said ratio is 1 µg of nucleic acid/0.3 nM of nanoparticles. In another preferred embodiment, said ratio is 3 µg of nucleic acid/0.3 nM of nanoparticles. In another preferred embodiment, said ratio is 3 µg of nucleic acid/0.3 nM of nanoparticles. Suitable concentrations of nanoparticles and nucleic acids which can be used according to the present method are detailed in the examples of the present application.

The second step of the fourth method of the invention comprises obtaining the SERS spectra of the first type and the second type of aggregates obtained in the first step of said method. The term "SERS spectrum" has been previously defined.

According to the present method the presence of a modified nucleotide at a predetermined position in a target sequence is determined by the SERS spectrum differences between:
(i) a first type of aggregates according to the invention, wherein said first type of aggregates are formed by metallic nanoparticles coated with a polycation and the target nucleic acid;
(ii) a second type of aggregates according to the invention, wherein said second type of aggregates are formed by metallic nanoparticles coated with a polycation and the control nucleic acid.

Once the SERS spectra from the first and second types of aggregates are obtained, the present method allows the detection of a modified nucleotide in a predetermined position in the target nucleic acid. Thus, if the SERS spectrum of the first type of aggregates and the SERS spectrum of the second types of aggregates are substantially identical, then the nucleotide at said determined position in the target nucleic acid is not modified. On the contrary, if the SERS spectrum of the first type of aggregates and the SERS spectrum of the second types of aggregates are different, then said predetermined position in the target nucleic acid is modified. The terms "SERS substantially identical" and "SERS different" have been defined in the context of the third method of the invention and equally apply to the present method.

In a preferred embodiment, the modification is detected in a predetermined nucleotide of cytosine or adenine.

The inventors have shown that the modification of a predetermined nucleotide within a target nucleic acid sequence results on changes on the band intensity and band shift in the SERS spectrum when compared with a SERS spectrum wherein said nucleotide is not modified. Thus, in a particular embodiment of the fourth method of the invention, the difference between the SERS of the first type of aggregates and the SERS spectrum of the second type of aggregates is selected from the group consisting of a decrease in the intensity of a band and a red shift of a band.

The term "redshift" as use herein, refers to any increase in the wavelength received by a detector compared with the wavelength emitted by the source. This increase in wavelength corresponds to a decrease in the frequency of the electromagnetic radiation. Redshift occurs when the electromagnetic radiation that is emitted from or reflected off of an object is shifted towards the red end of the electromagnetic spectrum.

In a particular and preferred embodiment, the fourth method of the invention further comprises a step of normalization of the SERS spectrum of the first and second types of aggregates using the peak height of the band at 1090 cm⁻¹.

In another particular embodiment, the modification of a predetermined nucleotide within a target nucleic acid sequence is a 5-methyl cytosine methylation. In a more particular embodiment, wherein said modification is a 5-methyl cytosine methylation, the difference between the spectrum of the first type of aggregates and the second types of aggregates is selected from the group consisting of:
(i) a decrease in intensity of the band at 599 cm⁻¹
(ii) a red shift and a decrease in intensity the band at 787 cm⁻¹,
(iii) an increase in the intensity of a band at 758 cm⁻¹,
(iv) a decrease in the intensity of a band at 1244 cm⁻¹,
(v) a decrease in the intensity of a band at 1288 cm⁻¹
(vi) an increase in the intensity of a band at 1218 cm⁻¹,
(vii) an increase in the intensity of a band at 1265 cm⁻¹,
(viii) an increase in the intensity of a band at 1315 cm⁻¹,
(ix) an increase in the intensity of a band at 1362 cm⁻¹ ; and
(x) a red shift and a decrease in intensity the band at 1653 cm⁻¹.

The term "a decrease in intensity" as used herein, refers to an at least 0.01-fold, 0.05-fold, 0.075-fold, 0.1-fold, 0.5-fold, 1-fold, 1.5-fold, 2-fold, 5-fold, 10-fold, 15-fold, 20-fold, 30-fold, 40-fold, 50-fold, 60-fold, 70-fold, 80-fold, 90-fold, or 100-fold decrease in the band intensity of the first type of aggregates with respect to said band intensity measured in the second type of aggregates. The term "a increase in intensity" as used herein, refers to an at least 0.01-fold, 0.05-fold, 0.075-fold, 0.1-fold, 0.5-fold, 1-fold, 1.5-fold, 2-fold, 5-fold, 10-fold, 15-fold, 20-fold, 30-fold, 40-fold, 50-fold, 60-fold, 70-fold, 80-fold, 90-fold, or 100-fold increase in the band intensity of the first type of aggregates with respect to said band intensity measured in the second type of aggregates.

In another particular embodiment, the modification of a predetermined nucleotide within a target nucleic acid sequence is a N6-methyl adenine methylation. In a more particular embodiment, wherein said modification is a N6-methyl adenine methylation, said difference between the spectrum of the first type of aggregates and the second types of aggregates is selected from the group consisting of:
(i) a red shift and an intensity decrease in the 731 cm⁻¹ band,
(ii) a decrease in the intensity of the 1509 cm⁻¹ band,
(iii) a shift and a intensity increase in the 1090 cm⁻¹ band,
(iv) an intensity decrease in the band at 1326 cm⁻¹,
(v) a redshift in the 1487 cm⁻¹ band and
(vi) a shift and an intensity decrease in the 1577 cm⁻¹ band.

In another particular embodiment of the fourth method of the invention, the target nucleic acid is a single stranded nucleic acid and the control nucleic acid is a double stranded nucleic acid. In a more particular embodiment of the invention, wherein the target nucleic acid is a single stranded nucleic acid, the first step of the present method is preceded by a step of contacting the target nucleic acid with a probe nucleic acid having a sequence which is fully complementary to the sequence of the target nucleic acid in the region comprising said predetermined position and wherein the control nucleic acid is a double stranded nucleic acid having a first strand the sequence of which has the same sequence as the target nucleic acid and wherein the nucleotide at said predetermined position is not modified and a second strand which is fully complementary with the target nucleic acid.

The terms "complementary" and "probe" have been defined in the context of the third method of the invention and are used herein with the same meaning. Suitable conditions for the hybridization between the singled stranded nucleic acid and the probe has been mentioned in the context of the third method of the invention and can be applied to this particular embodiment.

In another particular embodiment of the fourth method of the invention, the target nucleic acid, is a substantially isolated nucleic acid molecule. The term "substantially isolated nucleic acid" has been previously defined in the context of the third method of the invention and equally applies to this embodiment.

If desired, the target nucleic acid and/or the control nucleic acid can be subjected to a previous step of amplification (enrichment) before carry out the first step of the fourth method of the invention. Suitable methods for amplifying nucleic acids can be found on the context of the third method of the invention.

### Method for detecting the presence of a conjugate between a double stranded nucleic acid and a chemical in a sample

In another aspect, the invention relates to a method for detecting the presence of a conjugate between a double stranded nucleic acid and a chemical in a sample comprising double stranded nucleic acid molecules, hereinafter "the fifth method of the invention" comprising the steps of:
(i) contacting said sample with a population of metallic nanoparticles coated with a polycation, thereby forming an aggregate comprising metallic nanoparticles coated with a polycation and double stranded nucleic acid molecules stabilized by electrostatic interactions between the negative charges in the nucleic acid molecules and the positive charges of the polycation; and
(ii) obtaining the SERS spectrum of said sample,
wherein the presence in the spectrum of a one or more bands characteristic of the interaction between the nucleic acid and the chemical or of the chemical is indicative of the presence of said conjugate in the sample.

The terms "sample", "nucleic acid", "metallic nanoparticles", "electrostatic interactions", "polycation" and the particular and preferred embodiments have been previously defined and equally apply herein.

In the first step, the fifth method of the invention comprises contacting the sample of interest with a population of metallic nanoparticles coated with a polycation. Said first step must take place under conditions that allow the electrostatic interaction between the negative charges of the phosphate group of the nucleic acid, if present in the sample, and the positive charges of the polycation that coat the metallic nanoparticles. As has been explained above, said conditions under which the first step of the fifth method of the invention is carried out will be those conditions in which spontaneous nanoparticle aggregation does not occur, i.e., those conditions in which in absence of the sample to be detected the nanoparticles are in suspension and individualized. Suitable conditions that allow said electrostatic interaction have been detailed in the context of the second method of the invention. As has been previously mentioned, said conditions include those conditions wherein the molar ratio of nanoparticles to the double stranded double nucleic acid comprised in the sample must be adjusted in order to avoid large formation of unstable aggregates (high nanoparticle-to-nucleic acid ratios) or limited formation of stable aggregates (low nanoparticle-to-nucleic acid ratios

In a preferred embodiment, said ratio between metallic nanoparticles/nucleic acid refers to a molar ratio between said metallic nanoparticles/nucleic acid. In a preferred embodiment, said molar ratio is at least from about 1:1000 to about 1000:1, at least from about 1:900 to 900:1, at least from about 1:800 to 800:1, at least from about 1:700 to 700:1, at least from about 1:600 to 600:1, at least from about 1:500 to 500:1, at least from about 1:400 to 400:1, at least from about 1:300 to 300:1, at least from about 1:200 to 200:1, at least from about 1:100 to 100:1, at least from about 1:90 to 90:1, at least from about 1:80 to 80:1, at least from about 1:70 to 70:1, at least from about 1:60 to 60:1, at least from about 1:50 to 50:1, at least from about 1:40 to 40:1, at least from about 1:30 to 30:1, at least from about 1:20 to 20:1, at least from about 1:10 to 10:1, at least from about 1:5 to 5:1, at least from about 1:2 to 2:1. In another preferred embodiment said molar ratio is 1:100. In another preferred embodiment said molar ratio is 1:103 or more. In a more preferred embodiment said molar ratio is 1:1000. In another preferred embodiment said molar ratio is 1:166. In another preferred embodiment said molar ratio is 1:3. In another preferred embodiment said molar ratio is 1:833.

In another preferred embodiment, said ratio refers to the weight of said nucleic acid to molar concentration of said metallic nanoparticles. In this event, in a preferred embodiment the ratio is at least from about 0.1 to 1, at least from about 1 to 5, at least from about 5 to 10, at least from about 10 to 20, at least from about 20 to 30, at least from about 30 to 40, at least from about 40 to 50, at least from about 50 to 60, at least from about 60 to 70, at least from about 70 to 80, at least from about 80 to 90, at least from about 90 to 100, at least from about 100 to 110, at least from about 110 to 120, , at least from about 120 to 130, at least from about 130 to 140, , at least from about 140 to 150, at least from about 150 to 160, at least from about 160 to 170, at least from about 170 to 180, at least from about 180 to 190, at least from about 190 to 200. In a more preferred embodiment, said ratio is 1 µg of nucleic acid/0.3 nM of nanoparticles. In another preferred embodiment, said ratio is 3 µg of nucleic acid/0.3 nM of nanoparticles. In another preferred embodiment, said ratio is 3 µg of nucleic acid/0.3 nM of nanoparticles. Suitable concentrations of nanoparticles and nucleic acids which can be used according to the present method are detailed in the examples of the present application.

The second step of the fifth method of the invention comprises obtaining the SERS spectrum of said sample wherein the presence in the spectrum of a one or more bands characteristic of the interaction between the nucleic acid and the chemical is indicative of the present of said conjugated in the sample. The term "one or more bands characteristic of the interaction between the nucleic acid and the chemical" as used herein, refers to the specific bands which are not characteristic of the nucleic acid present in the sample and which are not characteristic of the chemical present in the sample but which are specific of the interaction between the reactive group of the chemical and the nucleic acid.

The second step of the fifth method of the invention also comprises determining the presence of a conjugate between a double stranded nucleic acid and a chemical in a sample by means of obtaining the SERS of the sample and detecting one or more bands characteristic of the chemical. To this end, the chemical which is present in the sample but which does not form a conjugate with the nucleic acid molecules present in said sample in the form of aggregate must be removed from the sample. Once the chemical has been removed from the sample and the SERS spectrum of the sample, which contains the aggregates, is obtained, the presence of one or more band characteristic of the chemical is indicative of the presence of said conjugate in said sample.

In a particular and preferred embodiment, the fifth method of the invention further comprises a step of normalization of the SERS spectrum using the peak height of the band at 1090 cm⁻¹.

In a particular embodiment of the fifth method of the invention, the nucleic acid is a double stranded DNA, the chemical is a platinum compound and the conjugate is an adduct. The term "platinum compound" as used herein refers to any chemical compound which comprises platinum atoms. In a more particular and preferred embodiment of the invention, said platinum compound is cisplatin.

The term "adduct" as used herein, refers to a product of a direct addition of two or more distinct molecules resulting in a single reaction product containing all atoms of all components. In a preferred embodiment, said conjugate is a DNA-adduct, in which the DNA is covalently bounded to the chemical. Illustrative and non-limitative examples of chemicals that forms DNA adduct are acetaldehyde, cisplatin, 7,12-dimethylbenz(a)antracene and malondialdehyde.

In another particular embodiment, the presence of a conjugate between a double stranded nucleic acid and a chemical in a sample, wherein the chemical is platinum is determined by the detection of one or more bands selected from the group consisting of:
(i) an intensity decrease in a band at 1487 cm⁻¹,
(ii) an intensity decrease in a band at about 1345 cm⁻¹,
(iii) a redshift and an intensity decrease in a band at about 1590 cm⁻¹,
(iv) an intensity decrease in a band at 1728 cm⁻¹,
(v) an intensity increase in a band at 1682 cm⁻¹,
(vi) an intensity increase in a band at 543 cm⁻¹,
(vii) an intensity increase in a band at 1325 cm⁻¹ and
(viii) an intensity increase in a band at 1509 cm⁻¹

The term "a decrease in intensity" as used herein, refers to an at least 0.01-fold, 0.05-fold, 0.075-fold, 0.1-fold, 0.5-fold, 1-fold, 1.5-fold, 2-fold, 5-fold, 10-fold, 15-fold, 20-fold, 30-fold, 40-fold, 50-fold, 60-fold, 70-fold, 80-fold, 90-fold, or 100-fold decrease in the band intensity of the of aggregates in presence of said chemical with respect to said band intensity measured in the aggregates in absence of said chemical. The term "an increase in intensity" as used herein, refers to an at least 0.01-fold, 0.05-fold, 0.075-fold, 0.1-fold, 0.5-fold, 1-fold, 1.5-fold, 2-fold, 5-fold, 10-fold, 15-fold, 20-fold, 30-fold, 40-fold, 50-fold, 60-fold, 70-fold, 80-fold, 90-fold, or 100-fold increase in the band intensity of the aggregates in presence of said chemical with respect to said band intensity measured in the aggregates in absence of said chemical.

In another particular embodiment of the fifth method of the invention, the nucleic acid is doubled stranded DNA, the chemical is a metallic ion and the conjugate is a coordination complex. The term "coordination complex" has been previously defined in the context of the first aspect of the invention and is used with the same meaning in the fifth aspect of the invention.

In a more particular embodiment, the metallic ion is Hg(II) and the complex is a coordination complex between said Hg(II) and a T:T duplex in the nucleic acid. In this event, in a still more particular embodiment of the fifth method of the invention, the presence of a conjugate between a double stranded nucleic acid and a chemical in a sample is determined by detection of one or more bands selected from the group consisting of:
(i) an intensity decrease of the band at 1580 cm⁻¹,
(ii) an intensity increase of the band at 1627 cm⁻¹,
(iii) an intensity decrease of the band at about 1305 cm⁻¹,
(iv) an intensity increase of the band at about 1239 cm⁻¹ and
(v) a downshift of the band at 787 cm⁻¹.

In another particular embodiment of the fifth method of the invention, the nucleic acid is double stranded DNA and the chemical is an intercalating compound. The term "intercalating compound", as used herein, refers to any chemical compound which inserts between the planar bases of the nucleic acid, such DNA. Examples of intercalating compounds are well known in the state of the art. In a particular embodiment, said intercalating compound is selected from the group consisting of DACA, proflavine, ethidium bromide, quinacrine, phenantridine, camptothecin, daunomycin, doxorubicin, nogalamycin, MPTQ, BPSQ, PPSQ, N-Hydroxybenzyl-isoxazolidinyl-PAHs, chartreusin, elsamicin A, HMPAP, 9-amino-acridine, bis.acridine, di-acridine, quinolone, bis-quinoline, acridine mustard, nitro-acridine, thieno-quinoline, flavonoids, anthracyclines, tamoxifen, N-Acetoxy-naphtamide, amino-fluorene, diolepoxides, aflatoxin B1 and methylene blue. In a preferred embodiment, said compound is methylene blue.

In another particular embodiment of the fifth method of the invention, the target nucleic acid is a substantially isolated nucleic acid molecule. The term "substantially isolated nucleic acid" has been previously defined in the context of the third method of the invention and equally applies to this embodiment.

### Method for determining the content of modified nucleotides in a target nucleic acid with respect to the total amount of nucleic acids in said sample

In another aspect, the invention relates to a method for determining the content of modified nucleotides in a target nucleic acid with respect to the total amount of nucleic acids in said sample, hereinafter, "the sixth method of the invention" comprising the steps of:
(i) contacting a population of metallic nanoparticles coated with a polycation separately with the target nucleic acid and with a reference nucleic acid, wherein said reference nucleic acid has the same sequence as the target nucleic acid and wherein none of the nucleotides contain said modification, thereby obtaining a first type of aggregates comprising the target nucleic acid and a second type of aggregates comprising the reference nucleic acid, wherein said aggregates are stabilized by electrostatic interactions between the negative charges in the nucleic acid and the positive charges of the polycation,
(ii) obtaining the SERS spectra of the first and second type of aggregates obtained in step (i),
(iii) obtaining the difference spectrum by subtracting from the spectrum of the second type of aggregates obtained in step (ii) the spectrum from the first type of aggregates and
(iv) determining the content of modified nucleotides in the sample as the value which corresponds to the value obtained by interpolation of the peak intensity of a band from the difference spectrum obtained in step (iii) within the peak intensities of said band in difference spectra obtained from a collection of samples having known contents of modified nucleotides.

The terms "nucleic acid", "metallic nanoparticles", "nucleotide modification", "aggregate", "polycation" and "electrostatic interactions" and the particular and preferred embodiments thereof have been previously defined and equally apply herein.

The first step of the sixth method of the invention comprises contacting a population of metallic nanoparticles coated with a polycation with the target nucleic acid. As has been explained above in the context of the second method of the invention, the result of putting in contact a nucleic acid with a metallic nanoparticle coated with a polycation is the formation of aggregates due to the electrostatic interaction between the negative charges of the phosphate groups of the nucleic acid and the positive charges of the polycation which coat the metallic nanoparticles. Thus, according to the first step of the sixth method of the invention, the contacting of a population of metallic nanoparticles coated with a polycation with said target nucleic acid results in the formation of a first type of aggregates.

The first step of the sixth method of the invention also comprises contacting said population of metallic nanoparticles coated with a polycation with a second sample, namely with a reference nucleic acid. Said reference nucleic acid is characterized in that it has the same sequence of the target nucleic acid and wherein none of the nucleotides are modified. The result of the electrostatic interaction between the negative charge of the reference nucleic acid and the positive charge of the polycation in the coats of said nanoparticles is the formation of a second type of aggregates. Suitable conditions that allow the electrostatic interactions between a nucleic acid and metallic nanoparticles coated with a polycation has been previously mentioned in the context of the second method of the invention. Suitable conditions that allow the formation of said first type of aggregates are preferred conditions that allow the formation of said second type of aggregates. As has been previously mentioned, the molar ratio of nanoparticles to the nucleic acid must be carefully adjusted in order to avoid large formation of unstable aggregates (high nanoparticle-to-nucleic acid ratios) or limited formation of stable aggregates (low nanoparticle-to-nucleic acid ratios). Thus, the ratio of the concentration of the metallic nanoparticles coated with a polycation to the concentration of the target nucleic acid and the ratio of the concentration of the metallic nanoparticles coated with a polycation to the concentration of the reference nucleic acid must be adjusted.

In a preferred embodiment, said ratio between metallic nanoparticles/nucleic acid refers to a molar ratio between said metallic nanoparticles/nucleic acid. In a preferred embodiment, said molar ratio is at least from about 1:1000 to about 1000:1, at least from about 1:900 to 900:1, at least from about 1:800 to 800:1, at least from about 1:700 to 700:1, at least from about 1:600 to 600:1, at least from about 1:500 to 500:1, at least from about 1:400 to 400:1, at least from about 1:300 to 300:1, at least from about 1:200 to 200:1, at least from about 1:100 to 100:1, at least from about 1:90 to 90:1, at least from about 1:80 to 80:1, at least from about 1:70 to 70:1, at least from about 1:60 to 60:1, at least from about 1:50 to 50:1, at least from about 1:40 to 40:1, at least from about 1:30 to 30:1, at least from about 1:20 to 20:1, at least from about 1:10 to 10:1, at least from about 1:5 to 5:1, at least from about 1:2 to 2:1. In another preferred embodiment said molar ratio is 1:100. In another preferred embodiment said molar ratio is 1:103 or more. In a more preferred embodiment said molar ratio is 1:1000. In another preferred embodiment said molar ratio is 1:166. In another preferred embodiment said molar ratio is 1:3. In another preferred embodiment said molar ratio is 1:833.

In another preferred embodiment, said ratio refers to the weight of said nucleic acid to molar concentration of said metallic nanoparticles. In this event, in a preferred embodiment the ratio is at least from about 0.1 to 1, at least from about 1 to 5, at least from about 5 to 10, at least from about 10 to 20, at least from about 20 to 30, at least from about 30 to 40, at least from about 40 to 50, at least from about 50 to 60, at least from about 60 to 70, at least from about 70 to 80, at least from about 80 to 90, at least from about 90 to 100, at least from about 100 to 110, at least from about 110 to 120, , at least from about 120 to 130, at least from about 130 to 140, , at least from about 140 to 150, at least from about 150 to 160, at least from about 160 to 170, at least from about 170 to 180, at least from about 180 to 190, at least from about 190 to 200. In a more preferred embodiment, said ratio is 1 µg of nucleic acid/0.3 nM of nanoparticles. In another preferred embodiment, said ratio is 3 µg of nucleic acid/0.3 nM of nanoparticles. In another preferred embodiment, said ratio is 3 µg of nucleic acid/0.3 nM of nanoparticles.

Suitable concentrations of nanoparticles and nucleic acids which can be used according to the present method are detailed in the examples of the present application.

Contacting the target nucleic acid with a population of metallic nanoparticles coated with a polycation according to the invention is done separately with respect to contacting said metallic nanoparticles with the reference nucleic acid sample. In preferred embodiments of the invention, the first step comprises contacting an aliquot of metallic nanoparticles coated with a polycation with the target nucleic acid to be detected giving rise to said first type of aggregates, and separately contacting another aliquot of the metallic nanoparticles coated with a polycation with the reference nucleic acid giving rise to said second type of aggregates, wherein said aliquot is different from the aliquot that is contacted with the target nucleic acid. Similar concentration of nanoparticles in said different aliquots is preferred. Methods for determining the concentration of nanoparticles are mentioned in the context of the second method of the invention.

The second step of the sixth method of the invention comprises obtaining the SERS spectra of the first type and the second type of aggregates obtained in the first step of said method. The term "SERS spectrum" has been previously defined.

The third step of the sixth method of the invention comprises obtaining the difference spectrum by subtracting from the spectrum of the second type of aggregates obtained in the second step of the present method the spectrum from the first type of aggregates. The spectrum subtraction can be done by using any algorithm known in the art. Illustrative examples of algorithms which can be used in the present invention include but are not limited to algorithms using linear convolution, causal filtering and/or dependent exponential averaging of the spectral subtraction gain function. The obtained bands correspond with those bands which are characteristic of the modified nucleotides.

The fourth step of the sixth method of the invention comprises determining the content of modified nucleotides in the sample as the value which corresponds to the value obtained by interpolation of the peak intensity of a band from the difference spectrum obtained in the third step of the present method within the peak intensities of said band in difference spectra obtained from a collection of samples having known contents of modified nucleotides. As has been shown in the fourth method of the invention, nucleotide modifications are associated with determined spectral changes (peak intensity and band shift); thus the person skilled in the art will identify the peaks of said difference spectrum with the corresponding nucleotide modification. As the person skilled in the art knows, in SERS, peak intensity depends on the number of molecules to the surface of the metallic nanoparticles. As the person skilled in the art knows, in SERS the concentration of a given sample (i.e. nucleic acid concentration) is proportional with the intensity of the peaks of the SERS spectrum of said sample. Thus, the person skilled in the art will understand that interpolation of a peak intensity of a band from a sample whose content is unknown within the peak intensity of a band from said sample having known content allows determining said unknown concentration. By way of illustration, the radiometric peak intensities obtained in a spectrum from a nucleic acid having known content of modified nucleotides can be represented versus known contents of nucleotides modifications. Then, the content of a modified nucleotide in the target nucleic acid can be determined by interpolating the peak intensity of the band corresponding to said nucleotide modification within the peak intensity corresponding to said band obtained from the nucleic acid having a known content of said modified nucleotide.

In a particular and preferred embodiment, the sixth method of the invention further comprises a step of normalization of the SERS spectrum using the peak height of the band at 1090 cm⁻¹.

In a particular embodiment of the sixth method of the invention, the nucleotide modification whose content is determined is selected from the group consisting of is selected from the group consisting of a 5-methyl Cytosine, a 5-hydroxymethyl Cytosine, a 5-X Cytosine, wherein X is Cl or Br, a N6-methyl Adenine, a 8-oxo Guanine, a cyclobutane pyrimidine dimer and a 6-4 photoproduct.

In another particular embodiment, the difference between the SERS spectrum of the first type of aggregates and the SERS spectrum of the second type of aggregates is selected from the group consisting of a decrease in the intensity of a band and a red-shift of a band. The terms "decrease in the intensity" and "red shift" have been previously defined".

In another particular embodiment, said nucleotide modification is a 5-methyl cytosine modification. In this event, in a still more particular embodiment, the peak intensity is determined using a band selected from:
(i) a decrease in intensity of the band at 599 cm⁻¹
(ii) a red shift and a decrease in intensity the band at 787 cm⁻¹,
(iii) an increase in the intensity of a band at 758 cm⁻¹,
(iv) a decrease in the intensity of a band at 1244 cm⁻¹,
(v) a decrease in the intensity of a band at 1288 cm⁻¹
(vi) an increase in the intensity of a band at 1218 cm⁻¹,
(vii) an increase in the intensity of a band at 1265 cm⁻¹,
(viii) an increase in the intensity of a band at 1315 cm⁻¹,
(ix) an increase in the intensity of a band at 1362 cm⁻¹ and
(x) a red shift and a decrease in intensity the band at 1653 cm⁻¹.

In another particular embodiment, said nucleotide modification is a N6-methyl adenine modification. In this event, in a still more particular embodiment, the peak intensity is determined using a band selected from:
(i) a red shift and an intensity decrease in the 731 cm⁻¹ band,
(ii) a decrease in the intensity of the 1509 cm⁻¹ band,
(iii) a shift and an intensity increase in the 1090 cm⁻¹ band,
(iv) an intensity decrease in the band at 1326 cm⁻¹,
(v) a redshift in the 1487 cm⁻¹ band and
(vi) a shift and an intensity decrease in the 1577 cm⁻¹ band.

### Method for determining the content of a nucleic acid conjugated to a chemical in a sample with respect to the total amount of nucleic acid in said sample

In another aspect, the invention relates to a method for determining the content of a nucleic acid conjugated to a chemical in a sample with respect to the total amount of nucleic acid in said sample, hereinafter "the seventh method of the invention", comprising the steps of:
(i) contacting a population of metallic nanoparticles coated with a polycation separately with the sample containing the conjugated nucleic acid and with a sample containing a reference nucleic acid, wherein said reference nucleic acid has the same sequence as the target nucleic acid and which is not conjugated to the chemical, thereby obtaining a first type of aggregates comprising the target nucleic acid and a second type of aggregates comprising the reference nucleic acid, wherein said aggregates are stabilized by electrostatic interactions between the negative charges in the nucleic acid and the positive charges of the polycation,
(ii) obtaining the SERS spectra of the first and second type of aggregates obtained in step (i),
(iii) obtaining the difference spectrum by subtracting from the spectrum of the second type of aggregates obtained in step (ii) the spectrum from the first type of aggregates and
(iv) determining the content of nucleic acid conjugated to the chemical in the sample with respect to the total amount of nucleic acid as the value which corresponds to the value obtained by interpolation of the peak intensity of a band from the difference spectrum obtained in step (iii) within the peak intensities of said band in difference spectra obtained from a collection of samples having known contents of conjugated nucleic acid.

The terms "nucleic acid", "metallic nanoparticles", "conjugate", "aggregate", "polycation" and "electrostatic interactions" and the particular and preferred embodiments thereof have been previously defined and equally apply herein.

The first step of the seventh method of the invention comprises contacting a population of metallic nanoparticles coated with a polycation with the sample containing the conjugated nucleic acid. As has been explained above the result of putting in contact a (conjugated) nucleic acid with a metallic nanoparticle coated with a polycation is the formation of aggregates due to the electrostatic interaction between the negative charges of the phosphate groups of the nucleic acid and the positive charges of the polycation which coat the metallic nanoparticles. Thus, according to the first step of the seventh method of the invention, the contacting of a population of metallic nanoparticles coated with a polycation with said conjugated nucleic acid results in the formation of a first type of aggregates.

The first step of the seventh method of the invention also comprises contacting said population of metallic nanoparticles coated with a polycation with a second sample, namely with a reference nuclei acid. Said reference nucleic acid is characterized in that it has the same sequence of the target nucleic acid and which is not conjugated to the chemical. The result of the electrostatic interaction between the negative charge of the reference nucleic acid and the positive charge of the polycation in the coats of said nanoparticles is the formation of a second type of aggregates. Suitable conditions that allow the electrostatic interactions between a nucleic acid and metallic nanoparticles coated with a polycation has been previously mentioned in the context of the second method of the invention. Suitable conditions that allow the formation of said first type of aggregates are preferred conditions that allow the formation of said second type of aggregates.

As has been mentioned above, contacting the sample containing the conjugated nucleic acid with a population of metallic nanoparticles coated with a polycation according to the invention is done separately with respect to contacting said metallic nanoparticles with the reference nucleic acid. In preferred embodiments of the invention, the first step comprises contacting an aliquot of metallic nanoparticles coated with a polycation with the sample containing the conjugated nucleic acid to be detected giving rise to said first type of aggregates, and separately contacting another aliquot of the metallic nanoparticles coated with a polycation with the reference nucleic acid giving rise to said second type of aggregates, wherein said aliquot is different from the aliquot that is contacted with the sample containing the conjugated nucleic. Similar concentration of nanoparticles in said different aliquots is preferred. Methods for determining the concentration of nanoparticles are mentioned in the context of the second method of the invention. As has been previously mentioned, the molar ratio of nanoparticles to the nucleic acid must be carefully adjusted in order to avoid large formation of unstable aggregates (high nanoparticle-to-nucleic acid ratios) or limited formation of stable aggregates (low nanoparticle-to-nucleic acid ratios). Thus, the ratio of the concentration of the metallic nanoparticles coated with a polycation to the concentration of the target nucleic acid and the ratio of the concentration of the metallic nanoparticles coated with a polycation to the concentration of the reference nucleic acid must be adjusted

In a preferred embodiment, said ratio between metallic nanoparticles/nucleic acid refers to a molar ratio between said metallic nanoparticles/nucleic acid. In a preferred embodiment, said molar ratio is at least from about 1:1000 to about 1000:1, at least from about 1:900 to 900:1, at least from about 1:800 to 800:1, at least from about 1:700 to 700:1, at least from about 1:600 to 600:1, at least from about 1:500 to 500:1, at least from about 1:400 to 400:1, at least from about 1:300 to 300:1, at least from about 1:200 to 200:1, at least from about 1:100 to 100:1, at least from about 1:90 to 90:1, at least from about 1:80 to 80:1, at least from about 1:70 to 70:1, at least from about 1:60 to 60:1, at least from about 1:50 to 50:1, at least from about 1:40 to 40:1, at least from about 1:30 to 30:1, at least from about 1:20 to 20:1, at least from about 1:10 to 10:1, at least from about 1:5 to 5:1, at least from about 1:2 to 2:1. In another preferred embodiment said molar ratio is 1:100. In another preferred embodiment said molar ratio is 1:103 or more. In a more preferred embodiment said molar ratio is 1:1000. In another preferred embodiment said molar ratio is 1:166. In another preferred embodiment said molar ratio is 1:3. In another preferred embodiment said molar ratio is 1:833.

In another preferred embodiment, said ratio refers to the weight of said nucleic acid to molar concentration of said metallic nanoparticles. In this event, in a preferred embodiment the ratio is at least from about 0.1 to 1, at least from about 1 to 5, at least from about 5 to 10, at least from about 10 to 20, at least from about 20 to 30, at least from about 30 to 40, at least from about 40 to 50, at least from about 50 to 60, at least from about 60 to 70, at least from about 70 to 80, at least from about 80 to 90, at least from about 90 to 100, at least from about 100 to 110, at least from about 110 to 120, , at least from about 120 to 130, at least from about 130 to 140, , at least from about 140 to 150, at least from about 150 to 160, at least from about 160 to 170, at least from about 170 to 180, at least from about 180 to 190, at least from about 190 to 200. In a more preferred embodiment, said ratio is 1 µg of nucleic acid/0.3 nM of nanoparticles. In another preferred embodiment, said ratio is 3 µg of nucleic acid/0.3 nM of nanoparticles. In another preferred embodiment, said ratio is 3 µg of nucleic acid/0.3 nM of nanoparticles. Suitable concentrations of nanoparticles and nucleic acids which can be used according to the present method are detailed in the examples of the present application. The second step of the seventh method of the invention comprises obtaining the SERS spectra of the first type and the second type of aggregates obtained in the first step of said method. The term "SERS spectrum" has been previously defined.

The third step of the seventh method of the invention comprises obtaining the difference spectrum by subtracting from the spectrum of the second type of aggregates obtained in the second step of the present method the spectrum from the first type of aggregates. The spectrum subtraction can be done by any suitable algorithm mentioned in the sixth method of the invention. Thus, the bands of the difference SERS spectrum correspond to those bands which are characteristic of the conjugated nucleic acid.

The fourth step of the seventh method of the invention comprises determining the content of nucleic acid conjugates to the chemical in the sample with respect to the total amount of nucleic acid as the value which corresponds to the value obtained by interpolation of the peak intensity of a band from the difference spectrum obtained in the third step of the present method within the peak intensities of said band in difference spectra obtained from a collection of samples having known contents of conjugates nucleic acid. The interpolation of the peak intensity of a band from the difference spectrum within the peak intensities of a sample wherein the parameter to be determined (i.e. the content of conjugated nucleic acid) is known has been explained in the context of the sixth method of the invention. By way of illustration, the radiometric peak intensities obtained in a spectrum wherein the content of conjugated nucleic acid is known can be represented versus known contents of conjugated nuclei acid. Then, the content of a conjugated nucleic acid with respect to the total amount of nucleic acid can be determined by interpolating the peak intensity of the band corresponding to said nucleic acid content within the peak intensity corresponding to said band obtained from the sample acid having a known content of said conjugated nucleic acid.

In a particular and preferred embodiment, the seventh method of the invention further comprises a step of normalization of the SERS spectrum using the peak height of the band at 1090 cm⁻¹.

In a particular embodiment of the seventh method of the invention, the nucleic acid wherein the content of conjugated nucleic acid is determined is double stranded DNA, the chemical is a platinum compound and the conjugate is an adduct. The term "platinum compound" and "adduct" have been previously defined.

In a more particular embodiment, the platinum compound is cisplatin. In a more particular embodiment the peak intensity is determined using a band selected from the group consisting of:
(i) an intensity decrease in a band at 1487 cm⁻¹,
(ii) an intensity decrease in a band at about 1345 cm⁻¹,
(iii) a redshift and an intensity decrease in a band at about 1590 cm⁻¹,
(iv) an intensity decrease in a band at 1728 cm⁻¹,
(v) an intensity increase in a band at 1682 cm⁻¹,
(vi) an intensity increase in a band at 543 cm⁻¹,
(vii) an intensity increase in a band at 1325 cm⁻¹ and
(viii) an intensity increase in a band at 1509 cm⁻¹

In another particular embodiment of the seventh method of the invention, the nucleic acid is double stranded DNA, the chemical is a metallic ion and the conjugate is a coordination complex. In a more particular embodiment, said metallic ion is Hg(II) and said complex coordination is a coordination complex between said Hg(II) and T:T duplex in the nucleic acid. In a more particular embodiment, the peak intensity is determined using a band selected from the group consisting of:
(i) an intensity decrease of the band at 1580 cm⁻¹,
(ii) an intensity increase of the band at 1627 cm⁻¹,
(iii) an intensity decrease of the band at about 1305 cm⁻¹,
(iv) an intensity increase of the band at about 1239 cm⁻¹ and
(v) a downshift of the band at 787 cm⁻¹.

In another particular embodiment of the seventh method of the invention, the nucleic acid is double stranded DNA and the chemical is an intercalating compound. In a more particular embodiment, said intercalating compound is selected from the group consisting of: a DACA, proflavine, ethidium bromide, quinacrine, phenantridine, camptothecin, daunomycin, doxorubicin, nogalamycin, MPTQ, BPSQ, PPSQ, N-Hydroxybenzyl-isoxazolidinyl-PAHs, chartreusin, elsamicin A, HMPAP, 9-amino-acridine, bis.acridine, di-acridine, quinolone, bis-quinoline, acridine mustard, nitro-acridine, thieno-quinoline, flavonoids, anthracyclines, tamoxifen, N-Acetoxy-naphtamide, amino-fluorene, diolepoxides, aflatoxin B1 and methylene blue.

### The present invention is also directed to:

[1]. An aggregate comprising metallic nanoparticles and nucleic acid molecules wherein each metallic nanoparticle is coated with a polycation and wherein said aggregate is formed by electrostatic interactions between the negative charges in the nucleic acid molecules and the positive charges of the polycation in the coats of said metallic nanoparticles, wherein said aggregate is not an aggregate of spermine-coated silver nanoparticles containing a single-stranded DNA modified with 5-FAM or Cy5 or a double stranded DNA modified with 5-FAM or Cy5.
[2]. The aggregate according to aspect [1] wherein the metal is silver, gold or a combination thereof.
[3]. The aggregate according to aspects [1] or [2] wherein the polycation is spermine, spermidine or putrescine.
[4]. The aggregate according to any of aspects [1] to [3] wherein the nucleic acid is selected from the group consisting of RNA, DNA, a double stranded nucleic acid, a single stranded nucleic acid, methylated DNA, a coordination complex of a nucleic acid and a metal, a coordination complex of a nucleic acid and a compound containing a metal and a complex of a nucleic acid and an intercalating organic dye.
[5]. The aggregate according to aspect [4] wherein the compound containing a metal is cisplatin.
[6]. A method for obtaining an aggregate according to any of aspects [1] to [5] comprising the steps of:
   (i) obtaining a population of metallic nanoparticles by contacting a salt of a metal and a hydrochloride of a polycation in the presence of a reducing agent under conditions adequate for the formation of the metallic nanoparticles coated with said polycation; and
   (ii) contacting the nanoparticles obtained in step (i) with a nucleic acid under conditions adequate for the formation of an aggregate formed by electrostatic interaction between a negatively charged nucleic acid and the positive charges of the polycation in the coat of said metallic nanoparticles.
[7]. The method according to aspect [6] wherein the reducing agent is a borohydride.
[8]. A method for detecting the presence of a nucleic acid in a sample, comprising the steps of:
   (i) contacting said sample with a population of metallic nanoparticles, wherein said metallic nanoparticles are coated with a polycation thereby forming aggregates of said metallic nanoparticles stabilized by electrostatic interactions between the negative charges in the nucleic acid and the positive charges of the polycation; and
   (ii) obtaining a SERS spectrum of the sample
   wherein an increase in the SERS spectrum of a band characteristic of a purine or pyrimidine base in a nucleic acid forming part of the aggregate is indicative of the presence of a nucleic acid in the sample.
[9]. The method according to aspect [8], further comprising a step of normalization of the SERS spectrum using the peak height of the band at 1090 cm⁻¹.
[10]. The method according to aspects [8] or [9] wherein
   (i) the band is selected from the group consisting of a band at about 503 cm⁻¹, at about 621 cm⁻¹, at about 665/677 cm⁻¹, at about 730 cm⁻¹, at about 752 cm⁻¹, at about 787 cm⁻¹, at about 1019 cm⁻¹, at about 1324 cm⁻¹, at about 1653 cm⁻¹, at about 2806 cm⁻¹ and at about 2967 cm⁻¹, then the nucleic acid is double stranded DNA,
   (ii) the band is selected from the group consisting of a band at about 512 cm⁻¹, about 686 cm⁻¹, at about 734 cm⁻¹, at about 793 cm⁻¹, at about 1029 cm⁻¹, at about 1199 cm⁻¹, at about 1329 cm⁻¹, at about 1643 cm⁻¹ and at about 2960 cm⁻¹, then the nucleic acid is single stranded DNA or
   (iii)the band is selected from the group consisting of a band at about 599 cm⁻¹, at about 1090 cm⁻¹, at about 1178 cm⁻¹, at about 1246/1264 cm⁻¹, at about 1354 cm⁻¹, at about 1376 cm⁻¹, at about 1421 cm⁻¹, at about 1487 cm⁻¹, at about 1509 cm⁻¹, at about 1528 cm⁻¹, at about 1577 cm⁻¹ and at about 1628 cm⁻¹, then the nucleic acid is single stranded RNA or double stranded RNA.
[11]. A method for detecting the presence of a given nucleotide at a predetermined position in a target nucleic acid comprising the steps of:
   (i) contacting a population of metallic nanoparticles coated with a polycation separately with the target nucleic acid and with a control nucleic acid having the same sequence as the target nucleic acid and having a known nucleotide at said predetermined position, thereby resulting in the formation of a first type of aggregates comprising the metallic nanoparticles and the target nucleic acid and a second type of aggregates comprising the metallic nanoparticles and the control nucleic acid,
   (ii) obtaining the SERS spectra of the first and second types of aggregates obtained in step (i); and
   wherein if the SERS spectrum of the first type of aggregates and the SERS spectrum of the second type of aggregates are substantially identical, then the nucleotide at said predetermined position in the target nucleic acid is the same as the known nucleotide or
   wherein if the SERS spectrum of the first type of aggregates and the SERS spectrum of the second type of aggregates are the SERS spectrum are different, then the nucleotide at said predetermined position is different from the known nucleotide.
[12].The method according to aspect [11] wherein the target nucleic acid is a single stranded nucleic acid, wherein step (i) is preceded by a step of contacting the target nucleic acid with a probe nucleic acid having a sequence which is fully complementary to the sequence of the target nucleic acid in the region comprising said predetermined position with the exception of the nucleotide at the predetermined position which contains a nucleotide different to the nucleotide complementary to said given nucleotide and wherein the control nucleic acid is a double stranded nucleic acid having a first strand the sequence of which has the same sequence as the target nucleic acid and having a known nucleotide at said predetermined position and a second strand which is fully complementary with the target nucleic acid.
[13]. The method according to aspect [12] wherein the position comprising the given nucleotide is located terminally in the double stranded nucleic acid.
[14]. The method according to any of aspects [11] or [13] wherein
   (i) if the difference between the spectra of the first and second types of aggregates is an increase in the intensity of a band selected from the group consisting of a band at 730 cm⁻¹, at 734 cm⁻¹, at 1224 cm⁻¹, at 1329 cm⁻¹, at 1508 cm⁻¹ and 1577cm⁻¹, then it is indicative that the nucleotide at said predetermined position is adenine,
   (ii) if the difference between the spectra of the first and second types of aggregates is an increase in the intensity of a band at 1577 cm⁻¹, then it is indicative that the nucleotide at said predetermined position is adenine or guanine,
   (iii)if the difference between the spectra of the first and second types of aggregates is an increase in the intensity of a band selected from the group consisting of a band at 621 cm⁻¹, at 665/677 cm⁻¹, at 686 cm⁻¹, at 1354 cm⁻¹, at 1487 cm⁻¹, then it is indicative that the nucleotide at said predetermined position is guanine,
   (iv)if the difference between the spectra of the first and second types of aggregates is an increase in the intensity of a band selected from the group consisting of a band at 787 cm⁻¹ and at 793 cm⁻¹, then it is indicative that the nucleotide at said predetermined position is cytosine or thymine,
   (v) if the difference between the spectra of the first and second types of aggregates is an increase in the intensity of a band selected from the group consisting of a band at 1178 cm⁻¹, at 1376 cm⁻¹, at 1643 cm⁻¹ and at 1653 cm⁻¹, then it is indicative that the nucleotide at said predetermined position is thymine.
   (vi) if the difference between the spectra of the first and second types of aggregates is an increase in the intensity of a band selected from the group consisting of a band at 1246/1264 cm⁻¹ and 1528 cm⁻¹, then it is indicative that the nucleotide at said predetermined position is cytosine or.
   (vii) if the difference between the spectra of the first and second types of aggregates is an increase in the intensity of a band selected from the group consisting of a band at 1274 cm⁻¹ and 1630 cm⁻¹, then it is indicative that the nucleotide at said predetermined position is uracil.
[15]. The method of any of aspects [11] to [14] further comprising a step of normalization of the SERS spectra using the peak height of the band at 1090 cm⁻¹.
[16]. The method according to any of aspects [11] to [15] wherein the target nucleic acid is a substantially isolated nucleic acid molecule.
[17]. A method for detecting the presence of a modified nucleotide at a predetermined position in a target nucleic acid comprising the steps of:
   (i) contacting a population of metallic nanoparticles coated with a polycation separately with the target nucleic acid and with a control nucleic acid having the same sequence as the target nucleic acid and wherein the predetermined position is not modified, thereby resulting in the formation of a first type of aggregates comprising the metallic nanoparticles and the target nucleic acid and a second type of aggregates comprising the metallic nanoparticles and the control nucleic acid,
   (ii) obtaining the SERS spectra of the first and second types of aggregates obtained in step (i) and
   wherein if the SERS spectrum of the first type of aggregates and the SERS spectrum of the second type of aggregates are substantially identical, then the nucleotide at said predetermined position is not modified or
   wherein if the SERS spectrum of the first type of aggregates and the SERS spectrum of the second type of aggregates are the SERS spectrum are different, then the nucleotide at said predetermined position is modified.
[18]. The method according to aspect [17] wherein the target nucleic as a single stranded nucleic acid, wherein step (i) is preceded by a step of contacting the target nucleic acid with a probe nucleic acid having a sequence which is fully complementary to the sequence of the target nucleic acid in the region comprising said determined position and wherein the control nucleic acid is a double stranded nucleic acid having a first strand the sequence of which has the same sequence as the target nucleic acid and wherein the nucleotide at said predetermined position is not modified and a second strand which is complementary with the target nucleic acid.
[19]. The method according to aspects [17] or [18] wherein said modification is selected from the group consisting of a 5-methyl Cytosine, a 5-hydroxymethyl Cytosine, a 5-X Cytosine, wherein X is Cl or Br, a N6-methyl Adenine, a 8-oxo Guanine, a cyclobutane pyrimidine dimer and a 6-4 photoproduct.
[20]. The method according to aspect [17] wherein the difference between the SERS spectrum of the first type of aggregates and the SERS spectrum of the second type of aggregates is selected from the group consisting of a decrease in the intensity of a band and a red-shift of a band.
[21]. The method according to aspect [20] wherein said modification is a 5-methyl cytosine methylation and the difference between the spectrum of the first type of aggregates and the second type of aggregates is selected from the group consisting of:
   (i) a decrease in intensity of the band at 599 cm⁻¹
   (ii) a red shift and a decrease in intensity the band at 787 cm⁻¹,
   (iii)an increase in the intensity of a band at 758 cm⁻¹,
   (iv) a decrease in the intensity of a band at 1244 cm⁻¹,
   (v) a decrease in the intensity of a band at 1288 cm⁻¹
   (vi) an increase in the intensity of a band at 1218 cm⁻¹,
   (vii) an increase in the intensity of a band at 1265 cm⁻¹,
   (viii) an increase in the intensity of a band at 1315 cm⁻¹,
   (ix) an increase in the intensity of a band at 1362 cm⁻¹ and
   (x) a red shift and a decrease in intensity the band at 1653 cm⁻¹,
[22]. The method according to aspect [20] wherein said modification is a N6-methyl adenine methylation and the difference between the spectrum of the first type of aggregates and the second type of aggregates is selected from the group consisting of the methylated nucleotide is adenine and the spectral change is selected from the group consisting of:
   (i) a red shift and an intensity decrease in the 731 cm⁻¹ band,
   (ii) a decrease in the intensity of the 1509 cm⁻¹ band,
   (iii)a shift and a intensity increase in the 1090 cm⁻¹ band,
   (iv) an intensity decrease in the band at 1326 cm⁻¹,
   (v) a redshift in the 1487 cm⁻¹ band and
   (vi) a shift and an intensity decrease in the 1577 cm⁻¹ band.
[23] The method of any of aspects [17] to [22] further comprising a step of normalization of the SERS spectra using the peak height of the band at 1090 cm⁻¹,
[24]. A method for detecting the presence of a conjugate between a double stranded nucleic acid and a chemical in a sample comprising double stranded nucleic acid molecules comprising the steps of:
   (i) contacting said sample with a population of metallic nanoparticles coated with a polycation, thereby forming an aggregate comprising metallic nanoparticles coated with a polycation and double stranded nucleic acid molecules stabilized by electrostatic interactions between the negative charges in the nucleic acid molecules and the positive charges of the polycation; and
   (ii) obtaining the SERS spectrum of said sample,
   wherein the presence in the spectrum of a one or more bands characteristic of the interaction between the nucleic acid and the chemical or of the chemical is indicative of the presence of said conjugate in the sample.
[25]. The method according to aspect [24] wherein the nucleic acid is double stranded DNA, the chemical is a platinum compound and the conjugate is an adduct.
[26]. The method according to aspect [25] wherein the platinum compound is cisplatin.
[27]. The method according to aspect [26] wherein the spectral change is selected from the group consisting of:
   (i) an intensity decrease in a band at 1487 cm⁻¹,
   (ii) an intensity decrease in a band at about 1345 cm⁻¹,
   (iii)a redshift and an intensity decrease in a band at about 1590 cm⁻¹,
   (iv)an intensity decrease in a band at 1728 cm⁻¹,
   (v) an intensity increase in a band at 1682 cm⁻¹,
   (vi) an intensity increase in a band at 543 cm⁻¹,
   (vii) an intensity increase in a band at 1325 cm⁻¹ and
   (viii)an intensity increase in a band at 1509 cm⁻¹
[28]. The method according to aspect [24] wherein the nucleic acid is double stranded DNA, the chemical is a metallic ion and the conjugate is a coordination complex.
[29]. The method according to aspect [28] wherein the metallic ion is HgII and the complex is a coordination complex between said HgII and a T:T duplex in the nucleic acid.
[30]. The method of aspect [29] wherein the spectral change is selected from the group consisting of:
   (i) an intensity decrease of the band at 1580 cm⁻¹,
   (ii) an intensity increase of the band at 1627 cm⁻¹,
   (iii)an intensity decrease of the band at about 1305 cm⁻¹,
   (iv) an intensity increase of the band at about 1239 cm⁻¹ and
   (v) a downshift of the band at 787 cm⁻¹,
[31]. The method according to aspect [24] wherein the nucleic acid is double stranded DNA and the chemical is an intercalating compound.
[32]. The method according to aspect [31] wherein the intercalating compound is selected from the group consisting of a DACA, proflavine, ethidium bromide, quinacrine, phenantridine, camptothecin, daunomycin, doxorubicin, nogalamycin, MPTQ, BPSQ, PPSQ, N-Hydroxybenzyl-isoxazolidinyl-PAHs, chartreusin, elsamicin A, HMPAP, 9-amino-acridine, bis.acridine, di-acridine, quinolone, bis-quinoline, acridine mustard, nitro-acridine, thieno-quinoline, flavonoids, anthracyclines, tamoxifen, N-Acetoxy-naphtamide, amino-fluorene, diolepoxides, aflatoxin B1 and methylene blue.
[33]. The method of any of aspects [24] to [32] further comprising a step of normalization of the SERS spectra using the peak height of the band at 1090 cm⁻¹,
[34]. The method according to any of aspects [17] to [33] wherein the target nucleic acid is a substantially isolated nucleic acid molecule.
[35]. A method for determining the content of modified nucleotides in a target nucleic acid comprising the steps of:
   (i) contacting a population of metallic nanoparticles coated with a polycation separately with the target nucleic acid and with a reference nucleic acid, wherein said reference nucleic acid has the same sequence as the target nucleic acid and wherein none of the nucleotides contain said modification, thereby obtaining a first type of aggregates comprising the target nucleic acid and a second type of aggregates comprising the reference nucleic acid, wherein said aggregates are stabilized by electrostatic interactions between the negative charges in the nucleic acid and the positive charges of the polycation,
   (ii) obtaining the SERS spectra of the first and second type of aggregates obtained in step (ii),
   (iii) obtaining the difference spectrum by subtracting from the spectrum of the second type of aggregates obtained in step (ii) the spectrum from the first type of aggregates and
   (iv) determining the content of modified nucleotides in the sample as the value which corresponds to the value obtained by interpolation of the peak intensity of a band from the difference spectrum obtained in step (iii) within the peak intensities of said band in difference spectra obtained from a collection of samples having known contents of modified nucleotides.
[36]. The method according to aspect [35] wherein said modification is selected from the group consisting of a 5-methyl Cytosine, a 5-hydroxymethyl Cytosine, a 5-X Cytosine, wherein X is Cl or Br, a N6-methyl Adenine, a 8-oxo Guanine, a cyclobutane pyrimidine dimer and a 6-4 photoproduct.
[37]. The method according to aspect [36] wherein the difference between the SERS spectrum of the first type of aggregates and the SERS spectrum of the second type of aggregates is selected from the group consisting of a decrease in the intensity of a band and a red-shift of a band.
[38]. The method according to aspect [36] wherein said modification is a 5-methyl cytosine methylation and the peak intensity is determined using a band selected from the group consisting of
   (i) a decrease in intensity of the band at 599 cm⁻¹
   (ii) a red shift and a decrease in intensity the band at 787 cm⁻¹,
   (iii) an increase in the intensity of a band at 758 cm⁻¹,
   (iv) a decrease in the intensity of a band at 1244 cm⁻¹,
   (v) a decrease in the intensity of a band at 1288 cm⁻¹
   (vi) an increase in the intensity of a band at 1218 cm⁻¹,
   (vii) an increase in the intensity of a band at 1265 cm⁻¹,
   (viii) an increase in the intensity of a band at 1315 cm⁻¹,
   (ix) an increase in the intensity of a band at 1362 cm⁻¹ and
   (x) a red shift and a decrease in intensity the band at 1653 cm⁻¹,
[39]. The method according to aspect [36] wherein said methylation is a N6-methyl adenine methylation and the peak intensity is determined using a band selected from the group consisting of
   (i) A red shift and an intensity decrease in the 731 cm⁻¹ band,
   (ii) A decrease in the intensity of the 1509 cm⁻¹ band,
   (iii) a shift and an intensity increase in the 1090 cm⁻¹ band,
   (iv) an intensity decrease in the band at 1326 cm⁻¹,
   (v) a redshift in the 1487 cm⁻¹ band and
   (vi) a shift and an intensity decrease in the 1577 cm⁻¹ band.
[40]. The method according to aspect of any of aspects [35] to [39] further comprising a step of normalization of the SERS spectra using the peak height of the band at 1090 cm⁻¹,
[41] A method for determining the content of a nucleic acid conjugated to a chemical in a sample with respect to the total amount of nucleic acid in said sample comprising the steps of:
   (i) contacting a population of metallic nanoparticles coated with a polycation separately with the sample containing the conjugated nucleic acid and with a sample containing a reference nucleic acid, wherein said reference nucleic acid has the same sequence as the target nucleic acid and which is not conjugated to the chemical, thereby obtaining a first type of aggregates comprising the target nucleic acid and a second type of aggregates comprising the reference nucleic acid, wherein said aggregates are stabilized by electrostatic interactions between the negative charges in the nucleic acid and the positive charges of the polycation,
   (ii) obtaining the SERS spectra of the first and second type of aggregates obtained in step (i),
   (iii)obtaining the difference spectrum by subtracting from the spectra of the second type of aggregates obtained in step (ii) the spectra from the first type of aggregates and
   (iv) determining the content of nucleic acid conjugated to the chemical in the sample with respect to the total amount of nucleic acid as the value which corresponds to the value obtained by interpolation of the peak intensity of a band from the difference spectrum obtained in step (iii) within the peak intensities of said band in difference spectra obtained from a collection of samples having known contents of conjugated nucleic acid.
[42]. The method according to aspect [38] wherein the nucleic acid is double stranded DNA, the chemical is a platinum compound and the conjugate is an adduct.
[43]. The method according to aspect [39] wherein the platinum compound is cisplatin.
[44]. The method according to aspect [40] wherein the peak intensity is determined using a band selected from the group consisting of:
   (i) an intensity decrease in a band at 1487 cm⁻¹,
   (ii) an intensity decrease in a band at about 1345 cm⁻¹,
   (iii) a redshift and an intensity decrease in a band at about 1590 cm⁻¹,
   (iv)an intensity decrease in a band at 1728 cm⁻¹,
   (v) an intensity increase in a band at 1682 cm⁻¹,
   (vi) an intensity increase in a band at 543 cm⁻¹,
   (vii) an intensity increase in a band at 1325 cm⁻¹ and
   (viii) an intensity increase in a band at 1509 cm⁻¹
[45]. The method according to aspect [41] wherein the nucleic acid is double stranded DNA, the chemical is a metallic ion and the conjugate is a coordination complex.
[46]. The method according to aspect [42] wherein the metallic ion is HgII and the complex is coordination complex between said HgII and a T:T duplex in the nucleic acid.
[47]. The method according to aspect [43] wherein the peak intensity is determined using a band selected from the group consisting of:
   (i) an intensity decrease in a band at 1580 cm⁻¹,
   (ii) an intensity increase in a band at 1627 cm⁻¹,
   (iii) an intensity decrease in of the band at about 1305 cm⁻¹,
   (iv) an intensity increase of the band at about 1239 cm⁻¹, and
   (v) a downshift of the band at 787 cm⁻¹,
[48]. The method according to aspect [44] wherein the nucleic acid is double stranded DNA and the chemical is an intercalating compound.
[49]. The method according to aspect [45] wherein the intercalating compound is selected from the group consisting of a DACA, proflavine, ethidium bromide, quinacrine, phenantridine, camptothecin, daunomycin, doxorubicin, nogalamycin, MPTQ, BPSQ, PPSQ, N-Hydroxybenzyl-isoxazolidinyl-PAHs, chartreusin, elsamicin A, HMPAP, 9-amino-acridine, bis.acridine, di-acridine, quinolone, bis-quinoline, acridine mustard, nitro-acridine, thieno-quinoline, flavonoids, anthracyclines, tamoxifen, N-Acetoxy-naphtamide, amino-fluorene, diolepoxides, aflatoxin B1 and methylene blue.
[50]. The method of any of aspects [41] to [49] further comprising a step of normalization of the SERS spectra using the peak height of the band at 1090 cm⁻¹

The following examples are provided as merely illustrative and are not to be construed as limiting the scope of the invention.

### EXAMPLES

### General methods

### Materials

All materials were obtained from Sigma Aldrich unless stated otherwise. DNA oligonucleotides were purchased from Eurofins MWG Operon. The oligonucleotide base sequences are shown in Table 1.

**Table 1**

| SEQ ID NO | Name | SEQUENCE |
|---|---|---|
| 1 | ss1 | CAT CGC AGG TAC CTG TAA GAG |
| 2 | ss2 | CAT CGC AGG TAC CTG TAA GA***A*** |
| 3 | ss3 | AAT CGC AGG TAC CTG TAA GAG |
| 4 | ss4 | CAT CGC AGG TA***A*** CTG TAA GAG |
| 5 | ss5 | CAT CGC AGG T***T***C CTG TA***T*** GAG |
| 6 | ss^{m}C | ^{m}CAT ^{m}CG^{m}C AGG TA^{m}C ^{m}CTG TAA GAG |
| 7 | ss^{m}A | C^{m}AT CGC ^{m}AGG T^{m}AC CTG T^{m}A^{m}A G^{m}AG |
| 8 | ssc | GTA GCG TCC ATG GAC ATT CTC |
| 9 | pA | AAA AAA AAA AAA AAA AAA AAA |
| 10 | pC | CCC CCC CCC CCC CCC CCC CCC |
| 11 | pT | TTT TTT TTT TTT TTT TTT TTT |
| 12 | pG | GGG GGG GGG GGG GGG GGG GGG |
| 13 | ssCG | CCG CGC CGC GCG CGC GGC GCGG |
| 14 | ssAT | AAT ATA ATA TAT ATA TTA TATT |

where ^{m}C and ^{m}A indicate 5-methyl Cytosine and N6-methyl Adenine residues, respectively. Stock solutions of each oligonucleotide were prepared in milli-Q water (final concentration about 4x10⁻⁴ M). Annealing was conducted by heating to 95°C for 10 minutes equimolar solutions of oligonucleotides ss1, ss2, ss3, ss4, ss5, ss^{m}C and ss^{m}A; and their complementary strand ssc in PBS buffer (0.3 M). This yielded the corresponding double-stranded DNA solutions ds1, ds2, ds3, ds4, ds5, ds^{m}C and ds^{m}A (final concentration 10⁻⁵ M) which were stored at -20 °C until required.

As reference samples, selected 21 base homopolymeric sequences (pA, pC, pT and pG) as well as 22 base self-complementary oligonucleotides (ssCG and ssAT) were selected. Annealing of ssCG and ssAT was conducted as indicated before and the resulting dsCG and dsAT samples (final concentration 10⁻⁵ M) were stored at -20 °C.

Deoxyribonucleic acid from calf thymus (Type XV, Activated, lyophilized powder) was purchased from Sigma Aldrich. Stock solution (320 µg/mL) was prepared in PBS 0.3 M and stored at -20 °C until required.

### Synthesis of Positively-charged Silver Nanoparticles.

Spermine coated-silver nanoparticles (AgNP@Sp) were prepared as previously reported (van Lierop et al., Chemical Communications 2012, 48, (66), 8192-8194). Briefly, 20 µL of a 0.5 M AgNO₃ solution were added to 10 ml of MilliQ water, followed by 7 µL of a 0.1 M spermine tetrahydrochloride (SpCl₄). The mixture was degassed for 30 min under N₂ flow, protected from light. Subsequently, under vigorous stirring, 250 µL of a freshly prepared NaBH₄ 0.01 M solution were added drop by drop to the mixture. Finally, the solution was gently stirred for 20 min. The colloidal suspension is composed of quasi-spherical nanoparticles of an average diameter of about 30 nm, with an extinction maximum centered at 391 nm. The final bulk pH is about 6. Glass vials were previously coated with PEI by an overnight immersion into an aqueous 0.2% v/v PEI solution, followed by extensive rinsing with Milli-Q water and N₂ drying.

### SERS measurements.

For SERS studies, 1 µL of dsDNA solutions (10⁻⁵ M) or 1 µL of ssDNA solutions (10⁻⁴ M) were mixed with 200 µL of AgNP@Sp ([NP] ca. 0.3 nM). The final analyte concentration in the sample was 5x10⁻⁸ M for dsDNAs and 5x10⁻⁷ M for ssDNAs. For genomic DNA (CTds), 5 µL of PBS (0.3 M) solutions (320 µg/mL) and its complexes were mixed with 200 µL of AgNP@Sp ([NP] ca. 0.3 nM). The final analyte concentrations in the samples were 7.8 µg/mL.

After the addition of the DNA, the colloids were left to equilibrate for 3 hour and resuspended by quick sonication before running the corresponding SERS measurements.

### DNA-hybridization study

Mixed ss and ds samples were prepared by combining different volumes of 10⁻⁵ M ssc and ds1 solutions (the final ssc+ds1 concentration in the mixtures was kept constant to 10⁻⁵ M) corresponding to the following molar ratios, R= [ds1]/([dsl]+[ssc]) = 0.011, 0.024, 0.041, 0.063, 0.091, 0.130, 0.189, 0.286, 0.474 and 1.

### Cisplatin (CP) cross-linking of dsDNA

A set of samples at different CP/ds1 molar ratio were prepared by mixing 80 µL of a 10-⁵M ds1 solution to 1 µL of fresh CP acqueous solutions at different concentrations. Similarly, 80 µL of the 320 µg/mL ctDNA solution were mixed with 1 µL of fresh CP aqueous solutions at different concentrations. The mixtures were left to stand overnight at 4° C and then investigated by SERS.

### Methylene blue (MB) intercalation in dsDNA

A set of samples at different MB/ds1 molar ratio were prepared by mixing 80 µL of a 10⁻⁵ M ds1 solution to 5 µL of MB ethanolic solutions at different concentrations. Similarly, 80 µL of the 320 µg/mL ctDNA solution were mixed with 5 µL of MB ethanolic solutions at different concentrations. The mixtures were left to stand overnight at 4° C and then investigated by SERS.

### T-Hg(II)-T metal-mediated base pair formation in T:T mismathed dsDNA

A set of samples at different Hg(II)/ds5 molar ratio were prepared by mixing 80 µL of a 10⁻⁵ M ds5 solution to 1 µL of fresh Hg(II) ethanolic solutions at different concentrations. The mixtures were left to stand overnight at 4° C and then investigated by SERS.

### Detection of methylated bases in dsDNA

Mixed ds samples were prepared by combining different volumes of 10⁻⁵ M solutions of ds1 and ds^{m}C/ds^{m}A (the final ds concentration in the mixtures was kept constant to 10⁻⁵ M).

### Instrumentation.

SERS experiments were conducted using a Renishaw InVia Reflex confocal microscope equipped with a high-resolution grating consisting of 1800 grooves/cm for visible wavelengths, additional band-pass filter optics, and a CCD camera. A 532 nm laser was focused onto the sample by a long-working distance objective (0.17 NA) and the spectra were typically acquired with an exposure time of 5x10 s. Baseline correction was applied to all spectra. Importantly, difference SERS spectra were calculated from the original no baseline-corrected spectra to avoid any generation of spectral artifacts. UV-vis spectra were recorded using a Thermo Scientific Evolution 201 UV-visible spectrophotometer.

### EXAMPLE 1: DNA hybridization: single vs double-stranded DNA sequences.

The addition of the negatively charged DNA sequences promotes the fast aggregation of positively-charged nanoparticles into long-term stable clusters in suspension *via* non-specific electrostatic interaction. The SERS spectra are acquired in colloidal suspensions under averaged bulk SERS regime yielding good-quality spectra with well-defined average band centers, bandwidths and relative intensities.

The average SERS spectra of two complementary single-stranded sequences (ss1 and ssc) and their corresponding double-helix structure (ds1) on AgNP@Sp colloids ([NP] ca. 0.3 nM) are shown in **Figure 1a****.** Vibrational assignment of dsDNA was based on literature references and comparative spectral analysis with homo- and bi-polymeric sequences (21-mer homopolymeric sequences of the four bases: pA, pC, pT and pG; and 22 base self-complementary oligonucleotides, ssCG and ssAT, yielding the corresponding dsCG and dsAT double-stranded sequences). In contrast with previous results on negatively charged colloids, when the individual ss units hybridize into the ds a large reshaping of their SERS spectra is observed **(****Fig. 1a**), influencing peak position, bandwidths and relative intensity. Notably, if ss 1 and ssc spectra show small changes in relative intensity that are associated with the base composition, the stacking and pairing of the nucleobases into the hybridized native structure is revealed by several characteristic features of the Raman melting profiles. Among others, the inventors highlight the marked intensity decrease and peak-shifting of the ring breathing modes (700-800 cm⁻¹) and the carbonyl stretching modes (1653 cm⁻¹), the latter one extremely sensitive to the disruption of Watson-Crick hydrogen bonding. One can also observe a general narrowing of the Raman features in the 1150-1600 cm⁻¹ spectral region, containing many overlapping bands mainly arising from in-plane vibrations of base residues. Such spectral modification can be ascribed to the adoption of a more rigid and well-defined conformation by the two individual ss when they self-organize into the rigid duplex geometry. The investigation of a set of solutions containing different ds1 and ssc molar ratios, R_{hybr} = [ds1]/([ds1]+[ssc]) **(****Fig. 1b**) shows a progressive shift of the ring breathing bands of adenine (from 734 cm⁻¹ to 730 cm⁻¹) when the DNA population is enriched with ds1. The spectral subtraction of the SERS spectra allows us to fully disclose the spectral alterations associated with changes in the DNA structure. In particular, the difference spectrum ds1-ssc (dotted line, **Fig. 1b****)** reveals a minimum at 724 cm⁻¹ and a maximum at 738 cm⁻¹, The corresponding ratiometric peak intensities I₇₂₄/I₇₃₈ were then selected to monitor the relative ds1/ssc populations in the samples, and plotted against R_{hybr} **(****Fig. 1c**). Data shows that ds sequences can be clearly identified within the mixture even when present at values < 1%, whereas SERS spectra are fully dominated by ds1 contributions for duplex populations ≥ 20%. Such result is consistent with the higher affinity of dsDNA to AgNP@Sp due to the larger availability of negatively-charged phosphate groups as compared to ssDNA. Linear correlation (r²>0.99) is observed in the 0.01-0.19 R_{hybr} range.

### EXAMPLE 2: Endogenous DNA modifications: Single-base mismatch

To test the possibility of detecting single-base mismatches in DNA duplexes, SERS spectra of the full-complementary ds1 and the heteroduplexes ds2, ds3 and ds4 were acquired and compared (Fig. 2). These heteroduplexes contain one adenine base, A, in place of: *(ds2)* one guanine, G, *(ds3)* one cytosine, C, (terminal position) and *(ds4)* one cytosine (internal position). Subtraction of the SERS spectra of ds1 from the other samples generates difference spectra containing vibrational signatures associated with the additional (positive features) and removed (negative features) nucleobase. Positive A bands emerge in all difference spectra at 730 and 1507 cm⁻¹, whereas negative G features are observed at ca. 620, 675 and 1354 cm⁻¹ in the ds2-ds1. In this case, negative bands also appear at 1487 and 1577 cm⁻¹, ascribed to purine modes (mainly G contribution) while the other purine feature at 1325 cm⁻¹ (mainly A contribution) does not suffer from a marked change in the relative intensity. These results, together with the lack of significant changes in SERS intensity of the pyrimidine bases, clearly indicate that the spectral alterations are ascribed to the A→G base-mismatch in the ds2. On the other hand, ds3-ds1 and ds4-ds1 difference spectra show very similar spectral patterns where, in addition to the positive A (730 and 1507 cm⁻¹) and the negative C (1250 and 1528 cm⁻¹) contributions, a consistent intensity increase of the purine bands (1325, 1487 and 1577 cm⁻¹) is observed. The pyrimidine ring breathing (787 cm⁻¹, C+T) also undergoes a drastic intensity decrease whereas thymine marker bands do not reveal significant alterations. These spectral changes can therefore be associated with the A→C base-mismatch in ds3 and ds4. Minor differences between ds3 and ds4 difference spectra **(****Fig. 2****)** can be ascribed to the different position of the base mismatch within the sequence.

### EXAMPLE 3: Endogenous DNA modifications: methylation of cytosine

The potential application of SERS in the identification of 5-methylated cytosine bases within helix structures was tested by acquiring the SERS spectrum of ds^{m}C and comparing it to its unmethylated analogous ds1. In the ds^{m}C, the cytosine nucleobases of one of the strands were all replaced by their 5-methylated counterparts (ss1 vs. ss^{m}C, see experimental methods). Figure 3a shows the SERS spectra of ds1 and ds^{m}C, as well as the corresponding difference spectra ds^{m}C-ds1. The introduction of the modified base induces marked spectral changes. First of all, a red-shift and relative intensity decrease of the pyrimidine ring breathing band (787 cm⁻¹) together with the appearance of a new weak feature (about758 cm⁻¹) was observed. Further, the difference spectrum reveals two negative bands located around 1244 and 1288 cm⁻¹, and three positive contributions broadly centered at 1218, 1265 and 1315 cm⁻¹ and a very strong sharp band at 1362 cm⁻¹, The cytidine ring band (1510 cm⁻¹) also red-shifts and decreases in intensity whereas the large red-shift of the broad carbonyl stretching band (1653 cm⁻¹) all in good agreement with the normal Raman studies (Gfrorer, A et al., Berichte Der BunsenGesellschaft-Physical Chemistry Chemical Physics 1991, 95, (7), 824-833). To examine the capability of SERS to quantitatively distinguish the percentage of ^{m}C bases within the sample, ds1+ds^{m}C mixtures at different molar ratios (while the total duplex concentration in the sample was kept constant to 5x10⁻⁸ M) was prepared. Details of the ring breathing spectral region are illustrated in **Fig. 3b****,** where the inventors highlight the progressive intensity decrease of the C+T ring breathing band at 787 cm⁻¹ upon increasing of the relative ^{m}C populations. Quantification of relative methylated base populations within the sample was investigated by monitoring the ratiometric peak intensities I₇₃₂/I₇₈₈ vs. the molar ratio R_{mC} = [^{m}C]/([C]+[^{m}C]) was monitored **(****Figure 3c****).** A linear correlation (r² >0.99) is observed in the whole investigated range, with a limit of detection of one 5-methylated cytosine nucleotide per 22 total cytosine bases in the sample.

### EXAMPLE 4: Endogenous DNA modifications: methylation of adenine

Analogously to ds^{m}C, the SERS spectra of ds1 and ds^{m}A was compared **(****Figure 4a****).** The spectral modifications of the SERS spectral profile of the normal DNA upon methylation of six of their eleven the adenine bases are striking. The adenine ring breathing band at 731 cm⁻¹ undergoes a drastic 11 cm-1 shift together with a notable intensity decrease, whereas the pyrimidine stretching of the adenine nucleobase at 1509 cm⁻¹ almost disappears from the spectrum. Furthermore, shifts of the phosphate stretching mode at 1090 cm⁻¹ were observed (together with a marked intensity increase); the v(CN) imidazole feature at 1487 cm⁻¹ and the 1577 cm⁻¹ band ascribed to base ring modes (mainly G+A). All these spectral changes are in good agreement with the normal Raman studies of the substitution of the methyl group into the N6 amino atom in adenosine nucleosides.(Mansy, S et al., Spectrochimica Acta Part a-Molecular and Biomolecular Spectroscope 1979, 35, (4), 315-329). As for ^{m}C, SERS study investigating the SERS ability to quantitatively distinguish the fraction of methylated adenine within the sample was studied by preparing different mixtures of ds1+ds^{m}A (the final overall concentration in the colloidal suspension was kept fixed at 5x10⁻⁸ M). Details of the ring breathing spectral region are illustrated in **Fig. 4b****.** In this case, the A ring breathing band at 730 cm⁻¹ suffers a dramatic intensity decrease and large peak red-shift as the molar ratio R_{mA} = [^{m}A]/([A]+[^{m}A]) value becomes larger. In **Figure 4c**, the ratiometric peak intensities I₇₄₃/I₇₃₀ is represented against the molar ratio R_{mA} = [^{m}A]/([A]+[^{m}A]), revealing a linear response in the investigated range (from 0.1 to 0.6 R_{mA}) with a r² >0.94 and a limit of detection of 1 N6-methylated adenine per 10 total adenine bases in the sample.

### EXAMPLE 5: Exogenous DNA modifications: Binding of the chemotherapeutic drug cisplatin

Since the initial discovery of its anticancer activity, cisplatin- (and its analogues) combination chemotherapy is the cornerstone of treatment of many cancers. The inorganic compound cisplatin (CP) forms covalent adducts with DNA, the most prevalent of which (>80%) is the 1,2-intrastrand crosslink between neighboring purine bases (preferably guanine *via* binding to the N7 atom) (Jamieson, E. R et al., Chemical Reviews 1999, 99, (9), 2467-2498). Such chemical binding lead to a large distortion of the duplex and a loss of helix stability which ultimately trigger cell apoptosis. Despite the great efficacy at treating specific kinds of cancers, CP suffers from several side-effects and intrinsic limitations (such acquired resistance of cells to the drug) which has fuelled an extensive amount of research aimed at developing new platinum-based drugs. However, only very few of these drugs candidates have entered clinical trials, possibly because their mechanism of action was neither fully understood nor used as the basis for their chemical design (Jamieson, E. R et al., Chemical Reviews 1999, 99, (9), 2467-2498).. Still, the resistance of cell to CP chemotherapy remains poorly understood even though it has been demonstrated that it is directly related to the extension of the DNA damages.

SERS monitoring of the CP binding to DNA sequences was restricted so far to thiolated sequences immobilized on gold nanoshell (Barhoumi, A.;et al, Journal of the American Chemical Society 2008, 130, (16), 5523-5529). Furthermore, in that study, the authors simply reported a reduction in SERS spectral reproducibility upon the addition of CP to dsDNA covalently bound to gold surfaces. In contrast, the deformation of the ds duplex induced by CP covalent binding is clearly and reproducibly reflected in the characteristic alteration of the SERS signal when AgNP@Sp colloids are employed as plasmonic substrates. The SERS spectra of the 21 base pair duplex ds1 and the corresponding adduct with CP are shown in Figure 5a. Characteristic spectral features of the covalent adduct formation mainly lie in the 1300-1600 cm⁻¹ region, such as the informative intensity decrease of the 1488 cm⁻¹ band, which has been associated to the binding of electrophilic agents to the N7 atom (Puppels, G. J et al, Biochemistry 1994, 33, (11), 3386-3395). Moreover, the marked intensity increase of the guanine contributions (ca. 1345 and ca. 1590 cm⁻¹), combined with the weakening of the guanine C=O stretching (1728 cm⁻¹) and the simultaneous enhanced intensity of the carbonyl contribution at 1682 cm⁻¹, has been associated with DNA pre-melting and/or denaturation at the guanine residues (Duguid, J. G et al., . Biophysical Journal 1996, 71, (6), 3350-3360).
Such characteristic spectral "fingerprints" of the CP-adduct do not only selectively inform about the type of complexation but can also be quantitatively correlated to the extension of such binding. **Figure 5b** illustrates SERS spectra in the 1430-1650 cm⁻¹ region obtained in the presence of increasing R_{CP} =[CP]/[ds1] molar ratios (the ds1 concentration was kept fixed throughout the whole study). These spectral changes were correlated quantitatively with CP concentration using as spectral marker the ratiometric peak intensity I₁₅₉₀/I₁₄₈₈, which was plotted against R_{CP} in **Figure 5c****.** Linear correlation (r²>0.98) is observed in the whole investigated range of R_{CP} = 0-50, with a detection limit close to the equimolar ratio (corresponding to 1 CP molecule per 21 base pairs).

### EXAMPLE 6: Exogenous DNA modifications: Intercalation of the organic dye methylene blue into DNA

The interaction of small molecule ligands with DNA sequences takes place also in a noncovalent manner, such as via intercalation of planar aromatic molecules into the space between two adjacent base pairs (Ferguson, L. R. et al., . Mutation Research-Fundamental and Molecular Mechanisms of Mutagenesis 2007, 623, (1-2), 14-23). The insertion of the DNA intercalating chemicals generally induces local structural changes to the DNA, including unwinding of the double helix and lengthening of the strand, which may lead to genotoxic effects as, for instance, frameshift mutagenesis (Ferguson, L. R. et al., . Mutation Research-Fundamental and Molecular Mechanisms of Mutagenesis 2007, 623, (1-2), 14-23). Methylene blue (MB) belongs to the class of phenothiazinium dyes and it has been employed in photodynamic therapy of tumors and other diseases. Additionally, MB has been also exploited in antimicrobial chemotherapy (particularly in the area of antimalarials), as well as a stain agent for DNA. Previous studies indicated that MB mainly binds dsDNA via intercalation of its aromatic moiety whereas the positive charge of MB would improve the DNA binding affinity by electrostatically interacting with the phosphate groups (Li, W. Y et al., Analytical Letters 2000, 33, (12), 2453-2464).

In contrast to CP, MB is an aromatic molecule with high Raman cross-section providing an intense SERS spectrum. In fact, the new intense features arising in the spectrum of the equimolar ds1+MB complex, which largely dominates the corresponding difference spectrum (ds1MB-ds1), are ascribed to the dye contribution **(****Fig. 6a****).** Importantly, the SERS profile of the intercalated MB markedly differs from that of the molecule directly adsorbed onto the colloidal nanoparticles **(****Fig. 6a****)** as revealed by the 3 nm up-shift of the strong C-C ring stretching (1626 cm⁻¹) (Xiao, G. N et al., Chemical Physics Letters 2007, 447, (4-6), 305-309).; the remarkable spectral reshaping in between 1380-1520 cm⁻¹ (including the band at 1432 cm⁻¹, ascribed to the asymmetric C-N stretching ; and the C-C stretching of the ring ¹¹ at 1477 and 1505 cm⁻¹) as well as the sharp band at 1040 cm⁻¹ associated with in-plane bending CH vibrations (Xiao, G. N et al., Chemical Physics Letters 2007, 447, (4-6), 305-309).. This spectral reshaping is associated with the specific electronic perturbation of the MB structure upon insertion within the aromatic nucleobases. Interestingly, when MB is left to interact with single-stranded sequences, the observed SERS spectra of the mixture shows MB contributions that largely matches those of the pure SERS of the intercalating agent. This spectroscopic evidence clearly suggests that MB molecules that loosely bind ssDNA via weak electrostatic interactions (Johnson, R. P et al., . Journal of the American Chemical Society 2012, 134, (34), 14099-14107) are free to directly interact with the metal surface producing the characteristic SERS signal of the MB-Ag surface complex. In contrast, in the presence of dsDNA, the effective sequestration of the MB molecules in the helix, sandwiched between aromatic heterocyclic base pairs by π-π stacking and dipole-dipole interactions, leads to a markedly different SERS profile. In this case, due to the strong and complex Raman spectrum of MB, a reliable spectral analysis of the binding agent-induced perturbations on the ds1 sequences cannot be longer performed by simple difference methods due to the large spectral overlapping.

As for CP, the inventors also monitored the SERS response of ds1+MB mixtures at different molar ratios by fixing the ds1 concentration and varying the MB amount (Fig. 6b). As a spectral marker, the inventors identified the ratiometric peak intensity I₁₅₉₀/I₁₄₈₈ between the intense MB band at 1626 cm⁻¹ and the ds1 band at 1577 cm⁻¹, which was plotted against the R_{MB} = [MB]/[ds1] molar ratios **(****Fig. 6c****),** revealing an excellent linear correlation (r²>0.99) with a limit of detection below R_{MB} = 1 (corresponding to ca. 1 dye molecule per 21 base pairs).

### EXAMPLE 7: Exogenous DNA modifications: DNA-metal coordination, formation of T-Hg(II)-T base pairs

Pyrimidine mismatched base pairs in DNA duplexes are known to selectively capture metal ions to form metal ion-mediated base pairs (Ono, A et al., Chemical Society Reviews 2011, 40, (12), 5855-5866). In particular, T:T mismatch pairs specifically capture Hg^{II} ions to form highly stable T-Hg^{II}-T pairs, a process where the dissociation of the imino protons of the thymine bases is followed by the formation of strong covalent N3-Hg bonds bridging the opposite pyridiminic bases (Ono, A et al., Chemical Society Reviews 2011, 40, (12), 5855-5866). The formation of T-Hg^{II}-T pairs within cells is also a bioprocess that was connected to mercury cytotoxicity (Clarkson, T. W et al, Critical Reviews in Toxicology 2006, 36, (8), 609-662). The affinity of thymine toward Hg^{II} ions has been largely exploited for the development of a multitude of DNA-based devices for mercury detection, including several SERS sensors. However, no label-free SERS study of the DNA-Hg^{II} interaction has been reported so far.
The SERS spectra of the ds2 heteroduplex, containing two T:T mismatch pairs before and after being exposed to an equimolar amount of HgCl₂ are shown in **Figure 7a****.** Analogously to what observed for the normal Raman study of T-Hg^{II}-T complex formation (Benda, L et al., Journal of Physical Chemistry A 2012, 116, (32), 8313-8320; Uchiyama, T et al., Nucleic Acids Research 2012, 40, (12), 5766-5774)., the spectral changes caused by the metal binding are mostly subtle but can be well highlighted by difference Raman spectroscopy. It is worth noticing, among others, the characteristic Raman marker band at ca. 1580 cm⁻¹, assigned to the C4=O4 stretching mode, that undergoes an unusually large downshift from ca. 1627 cm⁻¹ as a result of the reduction of the carbonyl bond order upon Hg^{II} coordination in the T:T mismatch. The disappearance of the weak feature at ca. 1305 cm⁻¹, associated with the deprotonation of the thymine N3 atom; the intensity increase of the ring deformation contribution at ca. 1239 cm⁻¹, and the 2 nm downshift and slight intensity increase of the ring breathing band at 787 cm⁻¹ are also consistent with the thymine-metal binding. Insertion of the mercury into the T:T mismatch pocket has also a direct influence on the neighboring base pairs (Ono, A.; et al., Chemical Society Reviews 2011, 40, (12), 5855-5866.), as indicated for instance by the significant change in the relative intensities between the Adenine band at ca. 1510 cm⁻¹ and the cytosine feature at ca. 1530 cm⁻¹ **(****Figure 7a****).** As an experimental control, the inventors performed the identical SERS study by replacing ds2 with the homopolymeric thymine sequence, pT. The spectral changes revealed in this latter case largely match those observed for ds2 and are consistent with the thymine-metal binding (Benda, L et al., Journal of Physical Chemistry A 2012, 116, (32), 8313-8320; Uchiyama, T et al., Nucleic Acids Research 2012, 40, (12), 5766-5774). On the contrary, no changes in the SERS spectrum were observed when the ds2 heteroduplex was replaced with the fully complementary ds1 sequence.

The spectral changes illustrated in **Figure 7b** were correlated quantitatively with the molar ratio R_{Hg} = [Hg^{II}]/[ds2] using the ratiometric peak intensities I₇₈₀/I₇₉₅ as a spectral marker. The plot of I₇₈₀/I₇₉₅ *vs*. R_{Hg} (logarithmic scale) shows a detection limit of R_{Hg} ca. 0.05 (corresponding to one Hg^{II} ion per ten T:T mismatch pairs), with a saturation point at R_{Hg} ca. 10 and r²>0.93 in the 0.01-5 molar ratio range **(****Fig. 7c****).**

### EXAMPLE 8: Exogenous modifications of genomic DNA: binding of the chemotherapeutic drug cisplatin and intercalation of the organic dye methylene blue into DNA

High quality double-stranded DNA isolated from the thymus of calves (CTds) was used as a model genomic DNA for studying the interaction with CP and MB. Figure 8 shows the SERS spectra of CTds (7.8 µg/mL) and their complexes with CP and MB, as well as the corresponding digital subtracted spectra.

As for shorter dsDNA sequences, the relative CTds/NP molar ratio was optimized to generate intense, unvaried and reproducible averaged bulk SERS. This condition, in the case of short double helix sequences (21 base pairs), is satisfied in the range of ca. 0.3-6.3 µg of dsDNA per mL of colloids, whereas for the long genomic structure CTds the optimum concentration range lies in the interval between ca. 4- 26 µg/mL.

The characteristic spectral fingerprints of the CP-adduct and MB-intercalation well highlighted in the difference spectra nicely those observed for ds1 and, similarly, the corresponding spectral ratios I₁₅₉₀/I₁₄₈₈ (Fig. 8B-C, for CP adducts) and I₁₆₂₆/I₁₅₇₇ (Fig. 8D-E, for MB complexes) show linear responses in the investigated ranges of nanomoles of ligand per mg of CTds (r² approximately equals to 0.99 and 0.98, respectively).

## Claims

1. An aggregate comprising metallic nanoparticles and nucleic acid molecules wherein each metallic nanoparticle is coated with a polycation and wherein said aggregate is formed by electrostatic interactions between the negative charges in the nucleic acid molecules and the positive charges of the polycation in the coats of said metallic nanoparticles, wherein said aggregate is not an aggregate of spermine-coated silver nanoparticles containing a single-stranded DNA modified with 5-FAM or Cy5 or a double stranded DNA modified with 5-FAM or Cy5.

2. The aggregate according to claim, 1 wherein the metal is silver, gold or a combination thereof.

3. The aggregate according to claims 1 or 2, wherein the polycation is spermine, spermidine or putrescine.

4. The aggregate according to any of claims 1 to 3, wherein the nucleic acid is selected from the group consisting of RNA, DNA, a double stranded nucleic acid, a single stranded nucleic acid, methylated DNA, a coordination complex of a nucleic acid and a metal, a coordination complex of a nucleic acid and a compound containing a metal and a complex of a nucleic acid and an intercalating organic dye.

5. A method for detecting the presence of a nucleic acid in a sample, comprising the steps of:
(i) contacting said sample with a population of metallic nanoparticles, wherein said metallic nanoparticles are coated with a polycation thereby forming aggregates of said metallic nanoparticles stabilized by electrostatic interactions between the negative charges in the nucleic acid and the positive charges of the polycation; and
(ii) obtaining a SERS spectrum of the sample
wherein an increase in the SERS spectrum of a band characteristic of a purine or pyrimidine base in a nucleic acid forming part of the aggregate is indicative of the presence of a nucleic acid in the sample.

6. The method according to claim 5 wherein
(i) the band is selected from the group consisting of a band at about 503 cm⁻¹, at about 621 cm⁻¹, at about 665/677 cm⁻¹, at about 730 cm⁻¹, at about 752 cm⁻¹, at about 787 cm⁻¹, at about 1019 cm⁻¹, at about 1324 cm⁻¹, at about 1653 cm⁻¹, at about 2806 cm⁻¹ and at about 2967 cm⁻¹, then the nucleic acid is double stranded DNA,
(ii) the band is selected from the group consisting of a band at about 512 cm⁻¹, about 686 cm⁻¹, at about 734 cm⁻¹, at about 793 cm⁻¹, at about 1029 cm⁻¹, at about 1199 cm⁻¹, at about 1329 cm⁻¹, at about 1643 cm⁻¹ and at about 2960 cm⁻¹, then the nucleic acid is single stranded DNA or
(iii) the band is selected from the group consisting of a band at about 599 cm⁻¹, at about 1090 cm⁻¹, at about 1178 cm⁻¹, at about 1246/1264 cm⁻¹, at about 1354 cm⁻¹, at about 1376 cm⁻¹, at about 1421 cm⁻¹, at about 1487 cm⁻¹, at about 1509 cm⁻¹, at about 1528 cm⁻¹, at about 1577 cm⁻¹ and at about 1628 cm⁻¹, then the nucleic acid is single stranded RNA or double stranded RNA.

7. A method for detecting the presence of a given nucleotide at a predetermined position in a target nucleic acid comprising the steps of:
(i) contacting a population of metallic nanoparticles coated with a polycation separately with the target nucleic acid and with a control nucleic acid having the same sequence as the target nucleic acid and having a known nucleotide at said predetermined position, thereby resulting in the formation of a first type of aggregates comprising the metallic nanoparticles and the target nucleic acid and a second type of aggregates comprising the metallic nanoparticles and the control nucleic acid,
(ii) obtaining the SERS spectra of the first and second types of aggregates obtained in step (i); and
wherein if the SERS spectrum of the first type of aggregates and the SERS spectrum of the second type of aggregates are substantially identical, then the nucleotide at said predetermined position in the target nucleic acid is the same as the known nucleotide or wherein if the SERS spectrum of the first type of aggregates and the SERS spectrum of the second type of aggregates are the SERS spectrum are different, then the nucleotide at said predetermined position is different from the known nucleotide.

8. The method according to claim 7 wherein
(i) if the difference between the spectra of the first and second types of aggregates is an increase in the intensity of a band selected from the group consisting of a band at 730 cm⁻¹, at 734 cm⁻¹, at 1224 cm⁻¹, at 1329 cm⁻¹, at 1508 cm⁻¹ and 1577cm⁻¹, then it is indicative that the nucleotide at said predetermined position is adenine,
(ii) if the difference between the spectra of the first and second types of aggregates is an increase in the intensity of a band at 1577 cm⁻¹, then it is indicative that the nucleotide at said predetermined position is adenine or guanine,
(iii) if the difference between the spectra of the first and second types of aggregates is an increase in the intensity of a band selected from the group consisting of a band at 621 cm⁻¹, at 665/677 cm⁻¹, at 686 cm⁻¹, at 1354 cm⁻¹, at 1487 cm⁻¹, then it is indicative that the nucleotide at said predetermined position is guanine,
(iv) if the difference between the spectra of the first and second types of aggregates is an increase in the intensity of a band selected from the group consisting of a band at 787 cm⁻¹ and at 793 cm⁻¹, then it is indicative that the nucleotide at said predetermined position is cytosine or thymine,
(v) if the difference between the spectra of the first and second types of aggregates is an increase in the intensity of a band selected from the group consisting of a band at 1178 cm⁻¹, at 1376 cm⁻¹, at 1643 cm⁻¹ and at 1653 cm⁻¹, then it is indicative that the nucleotide at said predetermined position is thymine.
(vi) if the difference between the spectra of the first and second types of aggregates is an increase in the intensity of a band selected from the group consisting of a band at 1246/1264 cm⁻¹ and 1528 cm⁻¹, then it is indicative that the nucleotide at said predetermined position is cytosine or.
(vii) if the difference between the spectra of the first and second types of aggregates is an increase in the intensity of a band selected from the group consisting of a band at 1274 cm⁻¹ and 1630 cm⁻¹, then it is indicative that the nucleotide at said predetermined position is uracil.

9. A method for detecting the presence of a modified nucleotide at a predetermined position in a target nucleic acid comprising the steps of:
(i) contacting a population of metallic nanoparticles coated with a polycation separately with the target nucleic acid and with a control nucleic acid having the same sequence as the target nucleic acid and wherein the predetermined position is not modified, thereby resulting in the formation of a first type of aggregates comprising the metallic nanoparticles and the target nucleic acid and a second type of aggregates comprising the metallic nanoparticles and the control nucleic acid,
(ii) obtaining the SERS spectra of the first and second types of aggregates obtained in step (i) and
wherein if the SERS spectrum of the first type of aggregates and the SERS spectrum of the second type of aggregates are substantially identical, then the nucleotide at said predetermined position is not modified or
wherein if the SERS spectrum of the first type of aggregates and the SERS spectrum of the second type of aggregates are the SERS spectrum are different, then the nucleotide at said predetermined position is modified.

10. The method according to claim 9 wherein said modification is selected from the group consisting of a 5-methyl Cytosine, a 5-hydroxymethyl Cytosine, a 5-X Cytosine, wherein X is Cl or Br, a N6-methyl Adenine, a 8-oxo Guanine, a cyclobutane pyrimidine dimer and a 6-4 photoproduct.

11. The method according to claim 10 wherein said modification is a 5-methyl cytosine methylation and the difference between the spectrum of the first type of aggregates and the second type of aggregates is selected from the group consisting of:
(i) a decrease in intensity of the band at 599 cm⁻¹
(ii) a red shift and a decrease in intensity the band at 787 cm⁻¹,
(iii) an increase in the intensity of a band at 758 cm⁻¹,
(iv) a decrease in the intensity of a band at 1244 cm⁻¹,
(v) a decrease in the intensity of a band at 1288 cm⁻¹
(vi) an increase in the intensity of a band at 1218 cm⁻¹,
(vii) an increase in the intensity of a band at 1265 cm⁻¹,
(viii) an increase in the intensity of a band at 1315 cm⁻¹,
(ix) an increase in the intensity of a band at 1362 cm⁻¹ and
(x) a red shift and a decrease in intensity the band at 1653 cm⁻¹,

12. The method according to claim 10 wherein said modification is a N6-methyl adenine methylation and the difference between the spectrum of the first type of aggregates and the second type of aggregates is selected from the group consisting of the methylated nucleotide is adenine and the spectral change is selected from the group consisting of:
(i) a red shift and an intensity decrease in the 731 cm⁻¹ band,
(ii) a decrease in the intensity of the 1509 cm⁻¹ band,
(iii) a shift and a intensity increase in the 1090 cm⁻¹ band,
(iv) an intensity decrease in the band at 1326 cm⁻¹,
(v) a redshift in the 1487 cm⁻¹ band and
(vi) a shift and an intensity decrease in the 1577 cm⁻¹ band.

13. A method for detecting the presence of a conjugate between a double stranded nucleic acid and a chemical in a sample comprising double stranded nucleic acid molecules comprising the steps of:
(i) contacting said sample with a population of metallic nanoparticles coated with a polycation, thereby forming an aggregate comprising metallic nanoparticles coated with a polycation and double stranded nucleic acid molecules stabilized by electrostatic interactions between the negative charges in the nucleic acid molecules and the positive charges of the polycation; and
(ii) obtaining the SERS spectrum of said sample,
wherein the presence in the spectrum of a one or more bands characteristic of the interaction between the nucleic acid and the chemical or of the chemical is indicative of the presence of said conjugate in the sample.

14. The method according to claim 13 wherein the spectral change is selected from the group consisting of:
(i) an intensity decrease in a band at 1487 cm⁻¹,
(ii) an intensity decrease in a band at about 1345 cm⁻¹,
(iii) a redshift and an intensity decrease in a band at about 1590 cm⁻¹,
(iv) an intensity decrease in a band at 1728 cm⁻¹,
(v) an intensity increase in a band at 1682 cm⁻¹,
(vi) an intensity increase in a band at 543 cm⁻¹,
(vii) an intensity increase in a band at 1325 cm⁻¹ and
(viii) an intensity increase in a band at 1509 cm⁻¹

15. The method according to claim 13 wherein the metallic ion is Hg(II) and the complex is a coordination complex between said Hg(II) and a T:T duplex in the nucleic acid.

16. The method of claim 15 wherein the spectral change is selected from the group consisting of:
(i) an intensity decrease of the band at 1580 cm⁻¹,
(ii) an intensity increase of the band at 1627 cm⁻¹,
(iii) an intensity decrease of the band at about 1305 cm⁻¹,
(iv) an intensity increase of the band at about 1239 cm⁻¹ and
(v) a downshift of the band at 787 cm⁻¹,
